# EUROPEAN PATENT APPLICATION

(11) **EP 4 722 383 A1**
(43) Date of publication of application: **08.04.2026**
(21) Application number: 23939107.1
(22) Date of filing: 11.07.2023
(51) Int. Cl.: C12Q 1/6844, C40B 50/18, C12Q 1/6806

(54) **METHOD FOR IMPROVING LOADING EFFICIENCY AND QUALITY OF NUCLEIC ACID NANOBALLS**

(30) Priority: 31.05.2023 WO PCT/CN2023/097511
(71) Applicant: MGI Tech Co., Ltd., Shenzhen, Guangdong 518083 (CN)
(72) Inventor: SU, Qiong, Shenzhen, Guangdong 518083 (CN); YANG, Jin, Shenzhen, Guangdong 518083 (CN); SONG, Chongyang, Shenzhen, Guangdong 518083 (CN)
(74) Representative: Boco IP Oy Ab
(86) International application number: PCT/CN2023/106790
(87) International publication number: WO 2024/244126

(57) **Abstract**

A method for adjusting the volume of nucleic acid nanoballs. By performing coagulation treatment on a nucleic acid (for example, adding a nucleic acid coagulating agent and/or adjusting the pH value to 3-4.5, so that positively charged counter ions are gathered around the nucleic acid, and negative charges on the surface of the nucleic acid are neutralized by cations by means of electrostatic bonding of anions and cations, thereby enabling the nucleic acid to coagulate), the shape of the nucleic acid nanoballs can be changed, the volume of the nucleic acid nanoballs can be adjusted, and the nucleic acid nanoballs can be compressed. The nucleic acid nanoballs obtained by the method (performing coagulation treatment on the nucleic acid nanoballs) are more compact, smaller in shape, and more uniform in size. The method can be used in any situation where the volume of the nucleic acid nanoballs needs to be adjusted (reduced) (such as the loading process of nucleic acid nanoballs (such as DNBs), and the sequencing process after loading).

## Description

### TECHNICAL FIELD

The present invention belongs to the field of nucleic acid sequencing, and in particular relates to a method for improving loading efficiency and quality of nucleic acid nanoballs.

### BACKGROUND

Microarray DNA NanoBall sequencing technology (DNBseq technology) is a type of high-throughput sequencing technology. The principle primarily involves amplifying the sequencing library via Rolling Circle Amplification (RCA) to obtain amplification products known as DNA NanoBalls (DNBs), which are then loaded onto a patterned microarray chip. Finally, an optimized Combinatorial Probe-Anchor Synthesis (cPAS) technology is used to achieve high-precision gene sequencing.

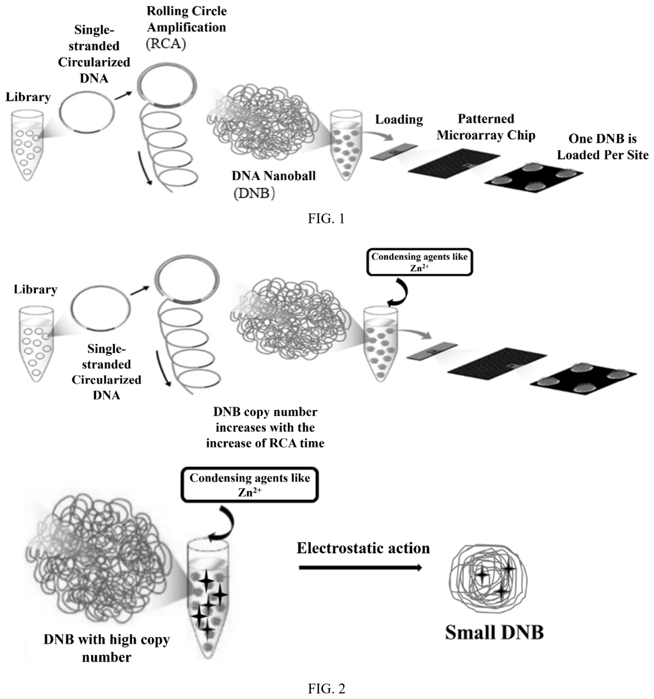

Taking the DNBseq technology used in MGI sequencers as an example: first, single-stranded circular DNA is used as a template, and RCA technology is used to obtain single-stranded circular DNBs. DNBs are loaded onto a patterned array chip and fixed in place through the gas-liquid system of the instrument. Finally, high-throughput sequencing is achieved under the enzymatic action and cPAS technology (FIG. 1).

The DNBseq technology requires the simultaneous fulfillment of the following three points to achieve high sequencing quality: 1) the DNB copy number is sufficiently high; 2) during loading, the DNBs are precisely and uniquely arranged on the microarray chip; 3) the generated optical signal is strong enough and easily distinguishable. However, problems still exist in practical applications: for example, when a sufficiently strong signal is required, it is often necessary to extend the RCA time to increase the copy number of the library. Within a certain time range, as the RCA time increases, the DNB copy number also increases. During gene sequencing process, a higher DNB copy number results in a stronger detection signal, which is beneficial for base calling and improves sequencing quality. However, an increased DNB copy number also leads to a larger DNB volume, leading to greater mutual interference between DNBs when loaded onto the patterned microarray chip, which results in issues such as more empty sites due to insufficient reactions, and non-uniform sizes of DNBs loaded onto the chip, thereby deteriorating the quality of gene sequencing. Therefore, it is necessary to find a method for increasing the DNB copy number without increasing the volume, so that it can be loaded onto the chip more effectively, thereby optimizing the microarray DNBseq technology to improve sequencing quality.

### SUMMARY

An objective of a first aspect of the present invention is to provide a method for adjusting a volume of nucleic acid nanoball (e.g., DNB).

An objective of a second aspect of the present invention is to provide a reagent for adjusting a volume of nucleic acid nanoball (e.g., DNB).

An objective of a third aspect of the present invention is to provide a method for improving loading efficiency and quality of a nucleic acid nanoball (e.g., DNB).

An objective of a fourth aspect of the present invention is to provide a sequencing method.

An objective of a fifth aspect of the present invention is to provide a kit.

In order to achieve the above objectives, the technical solution adopted by the present invention is as follows.

The first aspect of the present invention provides a method for adjusting a volume of nucleic acid nanoball (e.g., DNB), comprising subjecting a nucleic acid nanoball to a condensing treatment to obtain a volume-compressed nucleic acid nanoball;
a method for subjecting the nucleic acid nanoball to the condensing treatment comprises at least one of steps a1) and a2):
   a1) adding a nucleic acid condensing agent to a system comprising the nucleic acid nanoball;
   a2) adjusting a pH of the system comprising the nucleic acid nanoball to 3-4.5;
the nucleic acid condensing agent comprises at least one of a multivalent salt, a basic peptide, a basic protein, a polar solvent, a positively charged high molecular polymer, a polyamine, a polyammonium, and a lipid.

Preferably, the method for subjecting the nucleic acid nanoball to the condensing treatment comprises: step a1).

Preferably, the method for subjecting the nucleic acid nanoball to the condensing treatment comprises: step a2).

Preferably, the method for subjecting the nucleic acid nanoball to the condensing treatment comprises: steps a1) and a2).

Preferably, the basic protein comprises at least one of histone and protamine.

Preferably, the polar solvent comprises an alcohol.

Preferably, the positively charged high molecular polymer comprises at least one of polyhistidine, polylysine, polyarginine, and polyornithine.

Preferably, the polyamine comprises at least one of spermine, spermidine, and putrescine; further preferably comprising at least one of spermine and putrescine.

Preferably, the polyammonium comprises hexadimethrine bromide (polybrene).

Preferably, the lipid comprises at least one of 1,2-dioleoyl-3-trimethylammonium propane (DOTAP), 3β-[N-(N',N'-dimethylaminoethane)-carbamoyl]cholesterol hydrochloride/dioleoylphosphatidylethanolamine (DC-Chol/DOPE), 1,2-dioleoyl-sn-glycero-3-succinate (DOGS)/DOPE, and N,N,N-Trimethyl-2,3-bis((Z)-octadec-9-en-1-yloxy)propan-1-aminium chloride (DOTMA)/DOPE; further preferably comprising at least one of DOTAP and DC-Chol/DOPE.

Preferably, the nucleic acid condensing agent in a1) comprises at least one of a multivalent salt, a polyamine, and a lipid; further preferably, the nucleic acid condensing agent in a1) comprises a multivalent salt; still further preferably, the nucleic acid condensing agent in a1) comprises a multivalent metal salt.

Preferably, the multivalent metal salt comprises at least one of a divalent metal salt and a trivalent metal salt; further preferably, the multivalent metal salt comprises at least one of a zinc salt, a calcium salt, a magnesium salt, a copper salt, and a cerium salt (e.g., it may be ZnCl₂, ZnSO₄, CaCl₂, MgCl₂, CuCl₂·2H₂O, CeCl₃·7H₂O); still further preferably, the multivalent metal salt comprises at least one of a copper salt, a cerium salt, a calcium salt, and a zinc salt.

Preferably, a final concentration of the nucleic acid condensing agent in a1) in the system is 0.001-1000 mM; further preferably 1-1000 mM; still further preferably 10-100 mM; still further preferably 12-80 mM.

Preferably, the pH of the system in a2) is 4-4.5; further preferably 4.2-4.4.

The second aspect of the present invention provides a reagent for adjusting a volume of nucleic acid nanoball (e.g., DNB), the reagent for adjusting the volume of nucleic acid nanoball comprises at least one of b1) and b2):
b1) the reagent for adjusting the volume of nucleic acid nanoball comprises a nucleic acid condensing agent;
b2) a pH of the reagent for adjusting the volume of nucleic acid nanoball is 3-4.5;
the nucleic acid condensing agent comprises at least one of a multivalent salt, a basic peptide, a basic protein, a polar solvent, a positively charged high molecular polymer, a polyamine, a polyammonium, and a lipid.

The reagent for adjusting the volume of nucleic acid nanoball further comprises a loading buffer for nucleic acid nanoball, or a sequencing reagent.

Preferably, the reagent for adjusting the volume of nucleic acid nanoball comprises b1).

Preferably, the reagent for adjusting the volume of nucleic acid nanoball comprises b2).

Preferably, the reagent for adjusting the volume of nucleic acid nanoball comprises b1) and b2).

Preferably, the basic protein comprises at least one of histone and protamine.

Preferably, the polar solvent comprises an alcohol.

Preferably, the positively charged high molecular polymer comprises at least one of polyhistidine, polylysine, polyarginine, and polyornithine.

Preferably, the polyamine comprises at least one of spermine, spermidine, and putrescine; further preferably comprising at least one of spermine and putrescine.

Preferably, the polyammonium comprises hexadimethrine bromide (polybrene).

Preferably, the lipid comprises at least one of DOTAP, DC-Chol/DOPE, DOGS/DOPE, and DOTMA/DOPE; further preferably comprising at least one of DOTAP and DC-Chol/DOPE.

Preferably, the nucleic acid condensing agent in b1) comprises at least one of a multivalent salt, a polyamine, and a lipid; further preferably, the nucleic acid condensing agent in b1) comprises a multivalent salt; still further preferably, the nucleic acid condensing agent in b1) comprises a multivalent metal salt.

Preferably, the multivalent metal salt comprises at least one of a divalent metal salt and a trivalent metal salt; further preferably, the multivalent metal salt comprises at least one of a zinc salt, a calcium salt, a magnesium salt, a copper salt, and a cerium salt (e.g., it may be ZnCl₂, ZnSO₄, CaCl₂, MgCl₂, CuCl₂·2H₂O, CeCl₃·7H₂O); still further preferably, the multivalent metal salt comprises at least one of a copper salt, a cerium salt, a calcium salt, and a zinc salt.

Preferably, the pH of the reagent in b2) is 4-4.5; further preferably 4.2-4.4.

Preferably, a method for using the reagent for adjusting the volume of nucleic acid nanoball is as follows: mixing the reagent for adjusting the volume of nucleic acid nanoball with a nucleic acid nanoball to obtain a system for adjusting a volume of nucleic acid nanoball;
and the system satisfies at least one of c1) to c2):
c1) a final concentration of the nucleic acid condensing agent in the system for adjusting the volume of nucleic acid nanoball is 0.001-1000 mM (when the reagent for adjusting the volume of nucleic acid nanoball comprises b1) or simultaneously comprises b1) and b2)); and
c2) a pH of the system for adjusting the volume of nucleic acid nanoball is 3-4.5 (when the reagent for adjusting the volume of nucleic acid nanoball comprises b2) or simultaneously comprises b1) and b2)).

Preferably, the final concentration of the nucleic acid condensing agent in the system for adjusting the volume of nucleic acid nanoball in c1) is 1-1000 mM; further preferably 10-100 mM; still further preferably 12-80 mM.

Preferably, the pH of the system for adjusting the volume of nucleic acid nanoball in c2) is 4-4.5; further preferably 4.2-4.4.

Preferably, the loading buffer for nucleic acid nanoball (e.g., DNB) comprises at least one of citrate buffer, MES buffer solution, and Tris hydrochloride buffer.

Preferably, the sequencing reagent comprises at least one of a dNTPs mixture, a nucleic acid (e.g., DNA) polymerase mixture, an Multiple Displacement Amplification (MDA) reagent, and an MDA polymerase mixture; further preferably comprising a dNTPs mixture, a nucleic acid (e.g., DNA) polymerase mixture, an MDA reagent, and an MDA polymerase mixture.

The third aspect of the present invention provides a method for improving loading efficiency and quality of a nucleic acid nanoball (e.g., DNB), wherein the method for adjusting the volume of nucleic acid nanoball (e.g., DNB) according to the first aspect of the present invention is used during loading process of a nucleic acid nanoball.

Preferably, the method for improving loading efficiency and quality of the nucleic acid nanoball comprises the following steps:
subjecting a nucleic acid nanoball to a condensing treatment to obtain a volume-compressed nucleic acid nanoball;
loading the volume-compressed nucleic acid nanoball onto a solid support; and
a method for subjecting the nucleic acid nanoball to the condensing treatment is the method for subjecting the nucleic acid nanoball to the condensing treatment according to the first aspect of the present invention.

Preferably, the solid support comprises at least one of a latex bead, a dextran bead, a polystyrene surface, a polypropylene surface, a polyacrylamide gel, a gold surface, a glass surface, a chip, a sensor, an electrode, a silicon wafer; further preferably comprising a chip; still further preferably a high-density matrix chip.

Preferably, the nucleic acid nanoball (e.g., DNB) is prepared by Rolling Circle Amplification (RCA).

Preferably, the system comprising the nucleic acid nanoball in step a1) and/or step a2) further comprises a loading buffer for nucleic acid nanoball (e.g., DNB).

Preferably, the loading buffer for nucleic acid nanoball (e.g., DNB) is the loading buffer for nucleic acid nanoball according to the second aspect of the present invention.

Preferably, a rolling circle amplification time for the nucleic acid nanoball (e.g., DNB) is 20-120 min.

Preferably, the rolling circle amplification time for the nucleic acid nanoball (e.g., DNB) is 30-120 min.

Preferably, the rolling circle amplification time for the nucleic acid nanoball (e.g., DNB) is 40-120 min.

The fourth aspect of the present invention provides a sequencing method, comprising the following steps of:
1) adjusting a volume of a nucleic acid nanoball using the method according to the first aspect of the present invention;
2) sequencing to obtain sequence information carried by the nucleic acid nanoball.

Preferably, step 1) may be performed in any one or more sequencing working processes of the sequencing method.

Preferably, the sequencing working processes include but are not limited to: loading process of nucleic acid nanoball, sequencing process of nucleic acid nanoball.

Preferably, when step 1) is performed during the loading process of nucleic acid nanoball, the system comprising the nucleic acid nanoball further comprises a loading buffer for nucleic acid nanoball.

Preferably, the loading buffer for nucleic acid nanoball comprises at least one of citrate buffer, MES buffer solution, and Tris hydrochloride buffer.

Preferably, when step 1) is performed during the sequencing process of nucleic acid nanoball, the system comprising the nucleic acid nanoball further comprises a sequencing reagent.

Preferably, the sequencing reagent comprises at least one of a dNTPs mixture, a nucleic acid polymerase mixture, an MDA reagent, and an MDA polymerase mixture.

The fifth aspect of the present invention provides a kit comprising the reagent for adjusting the volume of nucleic acid nanoball (e.g., DNB) according to the second aspect of the present invention.

Preferably, the kit further comprises a preparation reagent for nucleic acid nanoball (e.g., DNB).

Preferably, the preparation reagent for nucleic acid nanoball (e.g., DNB) comprises at least one of TE buffer, nucleic acid nanoball (e.g., DNB) preparation buffer, a nucleic acid nanoball (e.g., DNB) polymerase mixture, and nucleic acid nanoball (e.g., DNB) termination buffer; further preferably comprising TE buffer, nucleic acid nanoball (e.g., DNB) preparation buffer, a nucleic acid nanoball (e.g., DNB) polymerase mixture, and nucleic acid nanoball (e.g., DNB) termination buffer.

Beneficial effects of the present invention are as follow.

The present invention provides a method for adjusting the volume of nucleic acid nanoball (e.g., DNB). By subjecting the nucleic acid (e.g., DNA) to a condensing treatment. The condensing treatment involves, for example, adding a nucleic acid condensing agent (such as a multivalent salt, a basic peptide, a basic protein, a polar solvent (e.g., an alcohol), a positively charged high molecular polymer, a polyamine, a polyammonium, or a lipid) and/or adjusting the pH to 3-4.5. It ,causes the accumulation of positively charged counterions around the nucleic acid (e.g., DNA), and through the electrostatic binding action between anions and cations, the negative charge on the surface of the nucleic acid (e.g., DNA) is neutralized by cations, thereby leading to a condensed morphological change the nucleic acid (e.g., DNA). Consequently, it can subsequently alter the morphology of the nucleic acid nanoball (e.g., DNB), adjust the volume of the nucleic acid nanoball (e.g., DNB), and compress the nucleic acid nanoball (e.g., DNB). The nucleic acid nanoball (e.g., DNB) obtained by this method (subjecting the nucleic acid nanoball to the condensing treatment) is more compact, smaller in morphology, and more uniform in size. This method can be used in any situation requiring adjustment (reduction) of the volume of nucleic acid nanoball (e.g., DNB),which can be in any one or more sequencing working processes, such as the loading process of nucleic acid nanoball (e.g., DNB), and the sequencing process after loading.

When this method is used in the loading process of nucleic acid nanoball (e.g., DNB), it can improve the loading efficiency and quality of the nucleic acid nanoball (e.g., DNB), making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality; at the same time, it can also enhance the detection signal to meet the requirements of long-read sequencing, specifically as follows:
For nucleic acid nanoballs (e.g., DNB) with the same RCA time, use of the method of the present invention for the loading process of nucleic acid nanoball (e.g., DNB) results in a lower Dup value compared to the existing technology (where the nucleic acid nanoball is not subjected to the condensing treatment, i.e., the method of the present invention is not used).

Furthermore, by limiting the rolling circle amplification time for the nucleic acid nanoball (e.g., DNB) to 30-120 min (increasing the RCA time), for large nucleic acid nanoballs (e.g., large DNB) obtained with increased RCA time, by using the method of the present invention for the loading process of nucleic acid nanoball (e.g., DNB), compared to the existing technology (where the nucleic acid nanoball is not subjected to the condensing treatment), since the morphology of the large nucleic acid nanoball is compressed, the loading efficiency and quality of the nucleic acid nanoball (e.g., DNB) is improved, making the arrangement of nucleic acid nanoballs on the chip more uniform and regular, the signal of large nucleic acid nanoballs at each site more concentrated, interference of adjacent sites reduced, and the Dup value lower. At the same time, after the volume of the large DNB is compressed, the interference caused by mutual competition for sites due to volume during the loading process is also reduced, and chip empty sites are reduced; the signal of large nucleic acid nanoballs is stronger and more concentrated, which is beneficial for base calling and improves sequencing quality. This solves the contradictory problem in the existing sequencing technology where the base signal to be enhanced by increasing the copy number is required, but the sequencing quality deteriorates, and can mitigate the problem of sequencing quality decline, which can meet the requirements of long-read sequencing.

Based on solving the contradictory problem of increasing the copy number to enhance the base signal but resulting in deteriorated sequencing quality, when the method of the present invention is used in the loading process of nucleic acid nanoball (e.g., DNB), it is possible to increase the RCA time while subjecting the obtained large nucleic acid nanoball to the condensing treatment, making the structure of the large nucleic acid nanoball (e.g., DNB) more compact and the size more uniform, improving the loading efficiency and quality of the nucleic acid nanoball (e.g., DNB), making the distribution loaded on the solid support (e.g., a chip) more uniform, with fewer empty sites and the size more uniform, the signal more concentrated, and less impact on adjacent sites, thereby lowering the Dup value and reducing empty sites on the chip. The combination of these two effects improves the sequencing quality of large nucleic acid nanoball (e.g., DNB), and has better sequencing quality compared to the nucleic acid nanoball (e.g., DNB) obtained with a short RCA time but without the condensing treatment.

Furthermore, use of the multivalent metal salt as the nucleic acid condensing agent can also improve the adsorption capacity of the solid support (e.g., a chip).

When this method is used in the sequencing process after loading, the volume of the nucleic acid nanoball (e.g., DNB) can be adjusted (e.g., to maintain the morphology of the DNB), further improving the sequencing quality.

### BRIEF DESCRIPTION OF DRAWINGS

FIG. 1 is a schematic diagram of the rolling circle amplification process for preparing DNB in the prior art and loading of the DNB onto a chip.
FIG. 2 is a schematic diagram of the method for loading nucleic acid nanoball of the present invention for subjecting DNB to a condensing treatment.
FIG. 3 is a result graph showing the effect of metal ions on Dup value and Q30 in Example 1: wherein the left vertical axis represents the Dup value, and the right vertical axis represents Q30.
FIG. 4 is a result graph showing the effect of DLBII pH on Dup value and Q30 in Example 2: wherein the left vertical axis represents the Dup value, and the right vertical axis represents Q30.
FIG. 5 is a result graph showing the effect of metal ion concentration on Dup value and Q30 in Example 3: wherein the left vertical axis represents the Dup value, and the right vertical axis represents Q30.

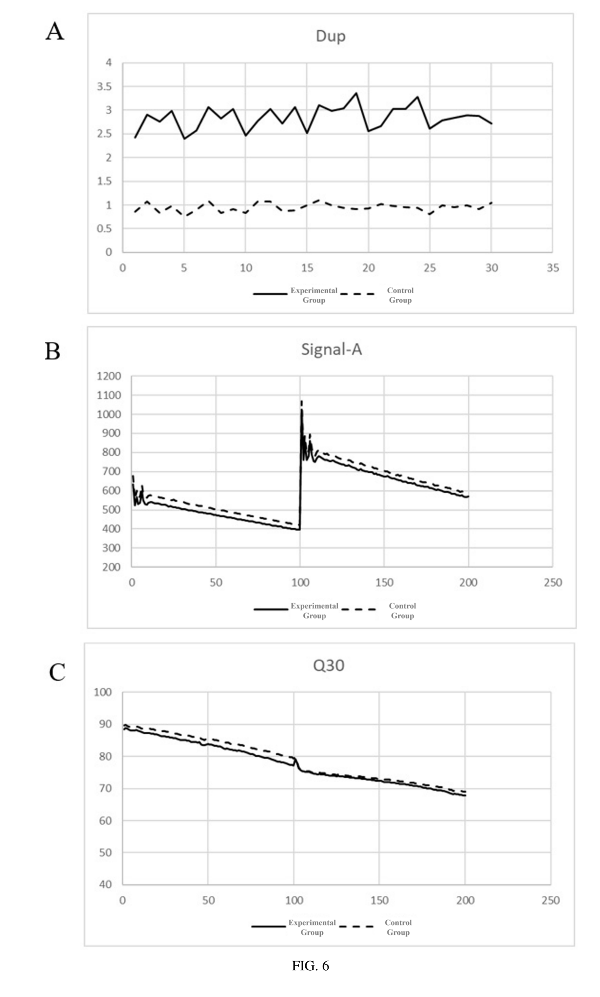

FIG. 6 is a graph of the paired-end PE100 sequencing results of the experimental group and the control group in Example 4: wherein A shows the Dup values of the paired-end PE100 sequencing results of the experimental group and the control group in Example 4 (the horizontal axis represents different FOVs, and the vertical axis represents Dup value); B shows the initial signal for base A (Signal-A) of the paired-end PE100 sequencing results of the experimental group and the control group in Example 4 (the horizontal axis represents sequencing cycle number, and the vertical axis represents Signal-A); C shows the Q30 of the paired-end PE100 sequencing results of the experimental group and the control group in Example 4 (the horizontal axis represents sequencing cycle number, and the vertical axis represents Q30).

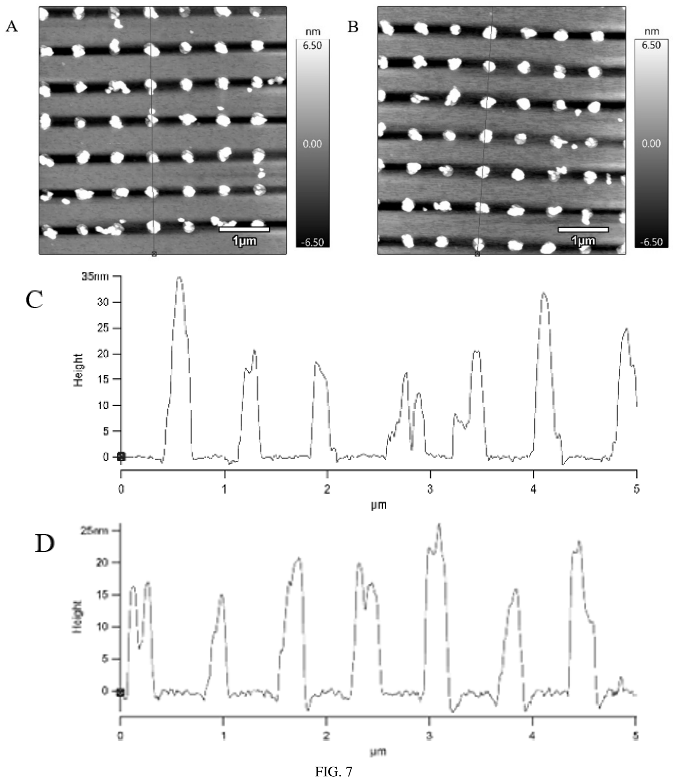

FIG. 7 is a result graph of the atomic force microscopy (AFM) characterization for the experimental group and the control group in Example 4: wherein A is an intuitive diagram of the atomic force microscopy (AFM) characterization for the control group (the scale bar represents 1µm); B is an intuitive diagram of the atomic force microscopy (AFM) characterization for the experimental group (the scale bar represents 1µm); C is a result graph of the DNB height of the control group (the horizontal axis represents a scanning range of 5µm, and the vertical axis represents DNB height); D is a result graph of the DNB height of the experimental group (the horizontal axis represents a scanning range of 5µm, and the vertical axis represents DNB height).
FIG. 8 is a result graph showing the effect of metal ion concentration on Dup value and Q30 in Example 5: wherein the left vertical axis represents the Dup value, and the right vertical axis represents Q30.

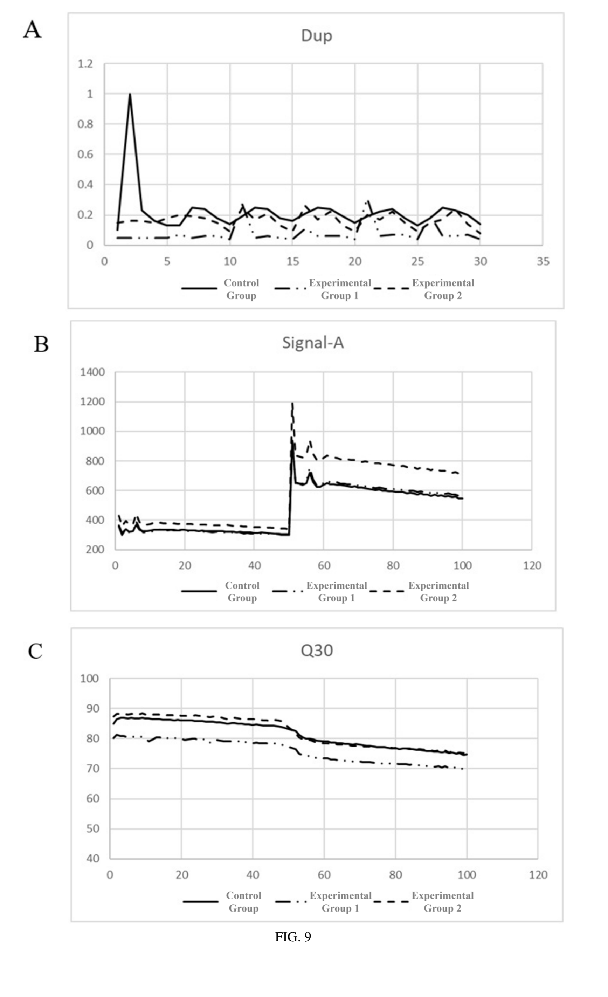

FIG. 9 is a graph of the paired-end PE50 sequencing results of the experimental group and the control group in Example 6: wherein A shows the Dup values of the paired-end PE50 sequencing results of the experimental group and the control group in Example 6 (the horizontal axis represents different FOVs, and the vertical axis represents Dup value); B shows the initial signal for base A (Signal-A) of the paired-end PE50 sequencing results of the experimental group and the control group in Example 6 (the horizontal axis represents sequencing cycle number, and the vertical axis represents Signal-A); C shows the Q30 of the paired-end PE50 sequencing results of the experimental group and the control group in Example 6 (the horizontal axis represents sequencing cycle number, and the vertical axis represents Q30).

### DETAILED DESCRIPTION

### Definition of terms

As used herein, the term "nucleic acid" can be any type of nucleic acid. For example, the nucleic acid can be deoxyribonucleic acid (DNA), ribonucleic acid (RNA), or an analog of DNA or RNA made from, for example, nucleotide analogs.

As used herein, the term "nucleic acid nanoball" generally refers to a complex or concatemer comprising multiple copies of a nucleic acid molecule. In certain typical schemes, these nucleic acid copies can be arranged one after another in a continuous linear chain of nucleotides. This tandem repeat structure, coupled with the single-stranded nature of the nucleic acid (e.g., DNA), appears to cause the nanoball to fold. It is readily understandable that the multiple copies of the nucleic acid molecule in the nucleic acid nanoball may each contain a linker sequence with a known sequence to facilitate its amplification or sequencing. The linker sequences of each nucleic acid molecule are usually identical, but may also be different. The nucleic acid nanoball includes, but is not limited to, a DNA nanoball (DNB), also referred to herein as DNB.

The nucleic acid nanoball can be generated using, for example, rolling circle replication (RCR) or rolling circle amplification (RCA). The RCR process has been used to prepare multiple consecutive copies of the M13 genome (Blanco et al., (1989) J Biol Chem 264:8935-8940). In this method, the nucleic acid is replicated via linear concatemerization. Those skilled in the art can find guidance on selecting conditions and reagents for RCR reactions in numerous references, including U.S. Patents US5,426,180, US5,854,033, US6,143,495, and US5,871,921, which are incorporated herein by reference in their entirety for all purposes, and in particular for all teachings related to the preparation of nucleic acid nanoball using RCR or other methods.

As used herein, the term "solid support" means any insoluble substrate or matrix to which a nucleic acid nanoball (e.g., DNB) can be attached, for example, latex beads, dextran beads, polystyrene surfaces, polypropylene surfaces, polyacrylamide gels, gold surfaces, glass surfaces, chips, sensors, electrodes, and silicon wafers. The surface of the solid support can be any desired shape, including, for example, planar, spherical, or porous suitable for a particular application; for instance, the solid support can be a planar glass surface. The solid support can be immobilized, for example, mounted inside a flow cell to allow interaction with a solution containing reagents. Alternatively, the solid support can also be movable to facilitate contact with solutions in different reaction vessels.

Typically, the solid support can comprise an array of sites arranged in a predetermined pattern, wherein each site can respectively attach or accommodate a nucleic acid nanoball (e.g., DNB). When the size of an attached nucleic acid nanoball (e.g., DNB) exceeds the maximum size that a single site can accommodate or exceeds the spacing between adjacent sites in the array, the nucleic acid nanoball (e.g., DNB) can occupy two or more sites, which can lead to a decrease in the utilization rate of the array sites and can affect sequencing efficiency and sequencing quality.

As used herein, the term "loading", when used in reference to a nucleic acid nanoball (e.g., DNB), means direct or indirect attachment to a solid support via covalent or non-covalent bonds. In certain embodiments of the present invention, the method of the present invention comprises immobilizing the nucleic acid nanoball (e.g., DNB) on the solid support via covalent attachment. However, generally, it is only required that under the conditions where the use of the solid support is desired (e.g., in applications requiring nucleic acid sequencing), the nucleic acid (e.g., nucleic acid nanoball, DNB) remains immobilized or attached to the solid support. Preferably, loading can occur by base-pairing hybridization or other means, such as the covalent attachment described above. Non-limiting examples of ways to attach the nucleic acid (e.g., nucleic acid nanoball (DNB)) to the solid support include nucleic acid hybridization, biotin-streptavidin binding, thiol binding, photoactivated binding, covalent binding, antibody-antigen interaction, physical confinement via hydrogel or other porous polymers, etc. Various exemplary methods for immobilizing the nucleic acid (e.g., nucleic acid nanoball (DNB)) on the solid support can be found in, for example, G. Steinberg-Tatman et al., Bioconjugate Chemistry 2006,17,841-848; Xu X. et al. Journal of the American Chemical Society 128(2006)9286-9287; U.S. Patents or Patent Applications US 5639603, US 5641658, US2010248991; International Patent Applications WO 2001062982, WO 2001012862, WO 2007111937, WO 0006770, which are incorporated herein by reference in their entirety for all purposes, in particular for all teachings related to the preparation of solid supports with nucleic acids immobilized thereon.

A key indicator for evaluating the quality of DNA nanoball (DNB) loading onto a chip is the size of Duplicate value (Dup value). In high-throughput sequencing, the Dup value refers to the proportion of duplicate reads in the total sequenced sequences. Duplicate reads mainly include two aspects: 1) the position and bases to which the sequenced reads are aligned on the genome are completely identical; 2) the direction of alignment to the reference genome is completely identical. Those satisfying both of the above aspects are considered as duplicate reads. The generation of Dup mainly includes the following aspects: 1) Dup inherent to the sample itself; 2) Dup introduced by amplification during library construction (i.e., PCR Dup); 3) Dup introduced by signal amplification before sequencing (the process of generating fluorescence signal acquisition unit); 4) optical Dup introduced during chip sequencing process. Theoretically, due to the complexity of the sample itself, the probability of Dup generating from the first two aspects is extremely low; Dup is more often generated by the latter two, i.e., Dup generated by optical resolution. When DNBs are loaded onto the chip, inconsistent DNB sizes can lead to incomplete reactions. For example, if a DNB signal is too strong (larger volume) or the loading position is offset, its signal coverage may exceed one DNB area, causing the sequences generated by sequencing of the "DNA nanoballs" at adjacent positions to be consistent, thus being mapped as two wells expressing the same signal, which is a type of optical Dup, namely NeighborDup(%). Therefore, it is necessary to balance the relationship between DNB copy number and Dup value to improve sequencing quality.

During the process of optimizing the microarray DNBseq technology, the inventors discovered that the Dup value is closely related to the DNB copy number. Longer RCA time results in more DNB copies, larger DNB size, and consequently a larger Dup value in the sequencing results. Conversely, shorter RCA time results in fewer DNB copies, smaller DNB size, and consequently a smaller Dup value in the sequencing results. However, there are certain problems with shortening the RCA time to lower the Dup value, that is, although the Dup value is small with short RCA time, the DNB copy number is small, leading to weak DNB signal during sequencing, which is not beneficial for identifying base sequence and cannot meet the requirements of long-read sequencing. Therefore, there is an urgent need to solve the problem where increasing RCA time increases DNB copy number but does not lead to an increase in the Dup value, so as to improve sequencing quality.

Furthermore, another key factor affecting sequencing quality is whether DNBs can be loaded uniformly onto the chip array when the DNBs are loaded onto a patterned microarray chip. During the process of optimizing the microarray DNBseq technology, the inventors discovered that when the DNB copy number increases, due to its larger morphology, when a large-volume DNB is loaded onto one site first, it becomes difficult for other large-volume DNBs to be loaded onto adjacent sites, resulting in more empty sites on the chip and a decline in sequencing quality. Therefore, it is also necessary to solve the problem where increasing RCA time leads to an increase in DNB copy number and an increase in the volume of DNB, which in turn causes more empty sites on the chip, so as to improve sequencing quality.

During their research, the inventors learned that nucleic acids (e.g., DNA) are long-chain polymers composed of deoxynucleotides arranged in a repeated sequence. In polar solutions, the bases and phosphates in their structures carry a large amount of negative charge. By subjecting the nucleic acid (e.g., DNA) to a condensing treatment (such as: adding a nucleic acid condensing agent such as a multivalent salt, a basic peptide, a basic protein, a polar solvent (e.g., an alcohol), a positively charged high molecular polymer, a polyamine, a polyammonium, a lipid) and/or adjusting the pH to 3-4.5, positively charged counterions are gathered around the nucleic acid (e.g., DNA). Through the electrostatic binding action between anions and cations, the negative charge on the surface of the nucleic acid (e.g., DNA) is neutralized by cations, thereby causing the morphology of the nucleic acid (e.g., DNA) to condense, which can subsequently alter the morphology of the nucleic acid nanoball (e.g., DNB), adjust the volume of the nucleic acid nanoball (e.g., DNB), and compress the nucleic acid nanoball (e.g., DNB).

The nucleic acid nanoball (e.g., DNB) obtained by this method (subjecting the nucleic acid nanoball to the condensing treatment) is more compact, smaller in morphology, and more uniform in size. This method can be used in any situation requiring adjustment (reduction) of the volume of nucleic acid nanoball (e.g., DNB) (which can be in any one or more sequencing working processes, such as the loading process of nucleic acid nanoball (e.g., DNB), and the sequencing process after loading).

When this method is used in the loading process of nucleic acid nanoball (e.g., DNB), it can improve the loading efficiency and quality of the nucleic acid nanoball (e.g., DNB), making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality. At the same time, it can also enhance the detection signal to meet the requirements of long-read sequencing. When this method is used in the sequencing process after loading, the volume of the nucleic acid nanoball (e.g., DNB) can be adjusted (e.g., to maintain the morphology of the DNB), further improving the sequencing quality.

Therefore, the inventors apply the condensing treatment (e.g., adding a nucleic acid condensing agent (such as a multivalent salt, a basic peptide, a basic protein, a polar solvent (e.g., an alcohol), a positively charged high molecular polymer, a polyamine, a polyammonium, a lipid) and adjusting the pH to 3-4.5) of the nucleic acid nanoballs (e.g., DNB), especially the nucleic acid nanoball with a high copy number (nucleic acid nanoball obtained by increasing RCA time) to any situation requiring adjustment (reduction) of the DNB volume (which can be in any one or more sequencing working processes, such as the loading process of nucleic acid nanoball (e.g., DNB), and the sequencing process after loading).

When this method is used in the loading process of nucleic acid nanoball (e.g., DNB), by improving the morphology of the nucleic acid nanoball (e.g., DNB), especially the nucleic acid nanoball with a high copy number (nucleic acid nanoball obtained by increasing RCA time, also known as large nucleic acid nanoball), the loading efficiency and quality of the nucleic acid nanoball are improved, thereby improving the loading effect onto the chip (lowering Dup value, reducing empty sites on the chip). That is, by increasing RCA time to obtain the nucleic acid nanoball with a high copy number (nucleic acid nanoball obtained by increasing RCA time, also known as large nucleic acid nanoball), and subjecting the nucleic acid nanoball (e.g., DNB) to the condensing treatment during loading to make the nucleic acid nanoball (e.g., DNB) smaller and more uniform in morphology, the nucleic acid nanoball with a high copy number (large nucleic acid nanoball) can be precisely and uniquely arranged on the microarray chip. At the same time, the signal of the large nucleic acid nanoball is stronger and more concentrated, which is beneficial for base calling and improves sequencing quality. This solves the contradictory problem in the existing sequencing technology where the base signal to be enhanced by increasing the copy number is required, but the sequencing quality deteriorates, thereby further improving the sequencing quality.

When this method is used in the sequencing process after loading of the nucleic acid nanoball (e.g., DNB), the volume of the nucleic acid nanoball (e.g., DNB) can be adjusted (e.g., to maintain the morphology of the DNB), further improving the sequencing quality.

The first aspect of the present invention provides a method for adjusting a volume of nucleic acid nanoball (e.g., DNB), comprising subjecting a nucleic acid nanoball to a condensing treatment to obtain a volume-compressed nucleic acid nanoball;
a method for subjecting the nucleic acid nanoball to the condensing treatment comprises at least one of steps a1) and a2):
   a1) adding a nucleic acid condensing agent to a system comprising the nucleic acid nanoball;
   a2) adjusting a pH of the system comprising the nucleic acid nanoball to 3-4.5;
the nucleic acid condensing agent comprises at least one of a multivalent salt, a basic peptide, a basic protein (e.g. histone, protamine), a polar solvent (e.g. an alcohol), a positively charged high molecular polymer (e.g. polyhistidine, polylysine, polyarginine, and polyornithine), a polyamine (e.g. spermine, spermidine, and putrescine), a polyammonium (e.g. polybrene (hexadimethrine bromide)), and a lipid (e.g. DOTAP, DC-Chol/DOPE, DOGS/DOPE, and DOTMA/DOPE).

During their research, the inventors learned that nucleic acids (e.g., DNA) are long-chain polymers composed of deoxynucleotides arranged in a repeated sequence. In polar solutions, the bases and phosphates in their structures carry a large amount of negative charge. By subjecting the nucleic acid (e.g., DNA) to a condensing treatment (e.g., adding a nucleic acid condensing agent such as a multivalent salt, a basic peptide, a basic protein, a polar solvent (e.g., an alcohol), a positively charged high molecular polymer, a polyamine, a polyammonium, a lipid) and/or adjusting the pH to 3-4.5, positively charged counterions are gathered around the nucleic acid (e.g., DNA). Through the electrostatic binding action between anions and cations, the negative charge on the surface of the nucleic acid (e.g., DNA) is neutralized by cations, thereby causing the morphology of the nucleic acid (e.g., DNA) to condense, which can subsequently alter the morphology of the nucleic acid nanoball (e.g., DNB), adjust the volume of the nucleic acid nanoball (e.g., DNB), and compress the nucleic acid nanoball (e.g., DNB), making the nucleic acid nanoball (e.g., DNB) more compact, smaller in morphology, and more uniform in size.

Preferably, the method for subjecting the nucleic acid nanoball to the condensing treatment comprises: step a1).

Preferably, the method for subjecting the nucleic acid nanoball to the condensing treatment comprises: step a2).

Preferably, the method for subjecting the nucleic acid nanoball to the condensing treatment comprises: steps a1) and a2).

Preferably, the nucleic acid condensing agent in step a1) comprises at least one of a multivalent salt, a polyamine, and a lipid; further preferably, the nucleic acid condensing agent in step a1) comprises a multivalent salt; still further preferably, the nucleic acid condensing agent in step a1) comprises a multivalent metal salt.

Preferably, the multivalent metal salt comprises at least one of a divalent metal salt and a trivalent metal salt; further preferably, the multivalent metal salt comprises at least one of a zinc salt, a calcium salt, a magnesium salt, a copper salt, and a cerium salt (e.g., it may be ZnCl₂, ZnSO₄, CaCl₂, MgCl₂, CuCl₂·2H₂O, CeCl₃·7H₂O); still further preferably, the multivalent metal salt comprises at least one of a copper salt, a cerium salt, a calcium salt, and a zinc salt.

Preferably, the polyamine comprises at least one of spermine and putrescine.

Preferably, the lipid comprises at least one of DOTAP and DC-Chol/DOPE.

Preferably, a final concentration of the nucleic acid condensing agent in a1) in the system is 0.001-1000 mM; further preferably 1-1000 mM; still further preferably 10-100 mM; still further preferably 12-80 mM.

Preferably, a final concentration of the zinc salt in the system is 1-1000 mM; further preferably 10-100 mM; still further preferably 20-80 mM.

Preferably, a final concentration of the calcium salt in the system is 1-1000 mM; further preferably 10-100 mM; still further preferably 12-20 mM.

Preferably, a final concentration of the copper salt in the system is 1-1000 mM; further preferably 10-100 mM; still further preferably 20-30 mM.

Preferably, a final concentration of the magnesium salt in the system is 1-1000 mM; further preferably 10-100 mM; still further preferably 20-30 mM.

Preferably, a final concentration of the cerium salt in the system is 1-1000 mM; further preferably 10-100 mM; still further preferably 20-30 mM.

Preferably, a final concentration of the polyamine in the system is 0.001-10 mM; further preferably 0.01-0.04 mM.

Preferably, a final concentration of the lipid in the system is 0.001-10 mM; further preferably 0.003-0.007 mM.

Preferably, the pH of the system in step a2) is 4-4.5; further preferably 4.2-4.4.

The second aspect of the present invention provides a reagent for adjusting a volume of nucleic acid nanoball (e.g., DNB),
the reagent for adjusting the volume of nucleic acid nanoball comprises at least one of b1) to b2):
   b1) the reagent for adjusting the volume of nucleic acid nanoball comprises a nucleic acid condensing agent;
   b2) a pH of the reagent for adjusting the volume of nucleic acid nanoball is 3-4.5;
the nucleic acid condensing agent comprises at least one of a multivalent salt, a basic peptide, a basic protein (e.g. histone, protamine), a polar solvent (e.g. an alcohol), a positively charged high molecular polymer (e.g. polyhistidine, polylysine, polyarginine, and polyornithine), a polyamine (e.g. spermine, spermidine, and putrescine), a polyammonium (e.g. polybrene (hexadimethrine bromide)), and a lipid (e.g. DOTAP, DC-Chol/DOPE, DOGS/DOPE, and DOTMA/DOPE).

By adding the nucleic acid condensing agent and/or adjusting the pH to 3-4.5, positively charged counterions are gathered around the nucleic acid (e.g., DNA). Through the electrostatic binding action between anions and cations, the negative charge on the surface of the nucleic acid (e.g., DNA) is neutralized by cations, thereby causing the morphology of the nucleic acid (e.g., DNA) to condense, which can subsequently alter the morphology of the nucleic acid nanoball (e.g., DNB), adjust the volume of the nucleic acid nanoball (e.g., DNB), and compress the nucleic acid nanoball (e.g., DNB), making the nucleic acid nanoball (e.g., DNB) more compact, smaller in morphology, and more uniform in size.

The reagent for adjusting the volume of nucleic acid nanoball further comprises a loading buffer for nucleic acid nanoball, or a sequencing reagent.

The nucleic acid nanoball (e.g., DNB) treated with the reagent for adjusting the volume of nucleic acid nanoball is more compact, smaller in morphology, and more uniform in size. This reagent for adjusting the volume of nucleic acid nanoball can be used in any situation requiring adjustment (reduction) of the DNB volume (which can be in any one or more sequencing working processes, such as the loading process of nucleic acid nanoball (e.g., DNB), and the sequencing process after loading).

When it is used in the loading process of nucleic acid nanoball (e.g., DNB) (i.e. when the reagent for adjusting the volume of nucleic acid nanoball further comprises a loading buffer for nucleic acid nanoball), the loading efficiency and quality of the nucleic acid nanoball (e.g., DNB) can be improved, making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality; at the same time, the detection signal can be enhanced to meet the requirements of long-read sequencing.

When it is used in the sequencing process after loading (i.e. when the reagent for adjusting the volume of nucleic acid nanoball further comprises a sequencing reagent), the nucleic acid nanoball (e.g., DNB) can be further adjusted (e.g., to maintain the morphology of the DNB), further improving the sequencing quality.

Preferably, the reagent for adjusting the volume of nucleic acid nanoball comprises b1).

Preferably, the reagent for adjusting the volume of nucleic acid nanoball comprises b2).

Preferably, the reagent for adjusting the volume of nucleic acid nanoball comprises b1) and b2).

Preferably, the nucleic acid condensing agent in b1) comprises at least one of a multivalent salt, a polyamine, and a lipid; further preferably, the nucleic acid condensing agent in b1) comprises a multivalent salt; still further preferably, the nucleic acid condensing agent in b1) comprises a multivalent metal salt.

Preferably, the multivalent metal salt comprises at least one of a divalent metal salt and a trivalent metal salt; further preferably, the multivalent metal salt comprises at least one of a zinc salt, a calcium salt, a magnesium salt, a copper salt, and a cerium salt (e.g., it may be ZnCl₂, ZnSO₄, CaCl₂, MgCl₂, CuCl₂·2H₂O, CeCl₃·7H₂O); still further preferably, the multivalent metal salt comprises at least one of a copper salt, a cerium salt, a calcium salt, and a zinc salt.

Preferably, the polyamine comprises at least one of spermine and putrescine.

Preferably, the lipid comprises at least one of DOTAP and DC-Chol/DOPE.

Preferably, the pH of the reagent in b2) is 4-4.5; further preferably 4.2-4.4.

Preferably, the reagent for adjusting the volume of nucleic acid nanoball further comprises instructions, wherein the instructions describe a method for using the reagent for adjusting the volume of nucleic acid nanoball.

Preferably, the method for using the reagent for adjusting the volume of nucleic acid nanoball is as follows: mixing the reagent for adjusting the volume of nucleic acid nanoball with a nucleic acid nanoball to obtain a system for adjusting a volume of nucleic acid nanoball;
and the system satisfies at least one of c1) to c2):
c1) a final concentration of the nucleic acid condensing agent in the system for adjusting the volume of nucleic acid nanoball is 0.001-1000 mM (when the reagent for adjusting the volume of nucleic acid nanoball comprises b1) or simultaneously comprises b1) and b2));
c2) a pH of the system for adjusting the volume of nucleic acid nanoball is 3-4.5 (when the reagent for adjusting the volume of nucleic acid nanoball comprises b2) or simultaneously comprises b1) and b2)).

Preferably, the final concentration of the nucleic acid condensing agent in the system for adjusting the volume of nucleic acid nanoball in c1) is 1-1000 mM; further preferably 10-100 mM; still further preferably 12-80 mM.

Preferably, a final concentration of the zinc salt in the system for adjusting the volume of nucleic acid nanoball is 1-1000 mM; further preferably 10-100 mM; still further preferably 20-80 mM.

Preferably, a final concentration of the calcium salt in the system for adjusting the volume of nucleic acid nanoball is 1-1000 mM; further preferably 10-100 mM; still further preferably 12-20 mM.

Preferably, a final concentration of the copper salt in the system for adjusting the volume of nucleic acid nanoball is 1-1000 mM; further preferably 10-100 mM; still further preferably 20-30 mM.

Preferably, a final concentration of the magnesium salt in the system for adjusting the volume of nucleic acid nanoball is 1-1000 mM; further preferably 10-100 mM; still further preferably 20-30 mM.

Preferably, a final concentration of the cerium salt in the system for adjusting the volume of nucleic acid nanoball is 1-1000 mM; further preferably 10-100 mM; still further preferably 20-30 mM.

Preferably, a final concentration of the polyamine in the system is 0.001-10 mM; further preferably 0.01-0.04 mM.

Preferably, a final concentration of the lipid in the system is 0.001-10 mM; further preferably 0.003-0.007 mM.

Preferably, the pH of the system for adjusting the volume of nucleic acid nanoball in c2) is 4-4.5; further preferably 4.2-4.4.

Preferably, the loading buffer for nucleic acid nanoball (e.g., DNB) comprises at least one of citrate buffer, MES buffer solution, and Tris hydrochloride buffer.

Preferably, a pH of the loading buffer for nucleic acid nanoball (e.g., DNB) is 4.6-5; further preferably 4.6-4.7.

Preferably, the sequencing reagent comprises at least one of a dNTPs mixture, a nucleic acid (e.g., DNA) polymerase mixture, an MDA reagent, and an MDA polymerase mixture; further preferably comprising a dNTPs mixture, a nucleic acid (e.g., DNA) polymerase mixture, an MDA reagent, and an MDA polymerase mixture.

Preferably, the dNTPs mixture comprises Tris(hydroxymethyl)aminomethane, hydrochloric acid, ethylenediaminetetraacetic acid, and chemically modified dNTPs.

Preferably, the chemical modifications include but are not limited to modifications of primary amino groups (e.g., 3'NH₂ C6, 5'NH₂ C6, 5'NH₂ C12, IntNH₂C6 dT, etc.), Int Uni-Link Amino modifications and derivatives thereof, carboxyl modifications and derivatives thereof, aldehyde modifications and derivatives thereof, acrylamide modifications and derivatives thereof, azide modifications and derivatives thereof (e.g., 5'Azide (N3), 3'Azide (N3), IntAzide-dT, etc.), alkyne modifications and derivatives thereof (e.g., 5'CHCH, Int CHCH-dT, 3'CHCH, etc.), dibenzocyclooctyne modifications and derivatives thereof (e.g., 5'DBCO, 3'DBCO, Int DBCO dT, etc.), maleimide modifications (e.g., 5'Maleimide, 3'Maleimide, etc.), biotin modifications (e.g., 5'Biotin, 3'Biotin, 3'Biotin-TEG, 5-Biotin-TEG, Triple Biotin, etc.), desthiobiotin modifications (e.g., 5'Desthiobiotin, 3'Desthiobiotin, etc.), SH/HS-SH/thiol modifications (e.g., 5'SH C6, 3'SH C6, 3'SH C3, 5'HS-SH C6, 3'HS-SH C3, 3'HS-SH C6, Int HS-SHC6, etc.), dithiol modifications and derivatives thereof (5'Dithiol, 3'Dithiol, lntDithiol, Int Dithiol dT, etc.), ferrocene modifications (e.g., 5'Ferrocene, 3'Ferrocene, Int Ferrocene dT, etc.), C3/C6 Spacer (5'C3 Spacer, 5'C6 Spacer, 3'C3 Spacer, 3'C6 Spacer, Int C6 Spacer, etc.), Spacer 9/Spacer18 modifications (5'Spacer 9, 5'Spacer 18, 3'Spacer 9, 3'Spacer 18, Int Spacer 9, Int Spacer18, etc.), PC Spacer/PC Linker photocleavable spacer, tetrahydrofuran modification, phosphorylation modifications (e.g., 5'P, 3'P, etc.), thio-modifications, phosphorothioate modifications, phosphorothioate modifications, 2-aminopurine modification, 5-bromodeoxyuridine modification, deoxyuridine modification, inverted dT/dG modification, dideoxycytidine modification, 5-methylcytosine deoxynucleoside modification, 5-hydroxymethyl dC modification, N6-methyladenine nucleotide modification, 5-aza-2-deoxycytidine modification, locked nucleic acid (LNA) modification, 2-O-methyl modified base modifications and derivatives thereof, RNA 2-fluoro RNA modification, pyrrole-deoxycytidine modification, digoxigenin modification, cholesterol modification, azobenzene modification, methylene blue modification, 8-oxo-7-hydrodeoxyguanosine modification and derivatives thereof, pyrene modification, Symmetric modification, ruthenium modification, etc.

Preferably, the nucleic acid (e.g., DNA) polymerase mixture comprises nucleic acid (e.g., DNA) polymerase and glycerol.

Preferably, the MDA reagent comprises deoxyribonucleoside triphosphates, dithiothreitol, dimethyl sulfoxide, sucrose, and glycerol.

Preferably, the MDA polymerase mixture comprises MDA polymerase and glycerol.

Preferably, the nucleic acid condensing agent and the loading buffer for nucleic acid nanoball/sequencing reagent exist independently or are mixed, i.e., the nucleic acid condensing agent may exist independently of the loading buffer for nucleic acid nanoball/sequencing reagent, or may be present in the loading buffer for nucleic acid nanoball/sequencing reagent.

The aforementioned dNTPs mixture, nucleic acid (e.g., DNA) polymerase mixture, MDA reagent, and MDA polymerase mixture are conventional reagents in the art and can be obtained commercially or prepared in-house.

The third aspect of the present invention provides a method for improving loading efficiency and quality of a nucleic acid nanoball (e.g., DNB), wherein the method for adjusting the volume of nucleic acid nanoball (e.g., DNB) according to the first aspect of the present invention is used during loading process of a nucleic acid nanoball.

Preferably, the method for improving loading efficiency and quality of the nucleic acid nanoball comprises the following steps:
subjecting a nucleic acid nanoball to a condensing treatment to obtain a volume-compressed nucleic acid nanoball;
loading the volume-compressed nucleic acid nanoball onto a solid support;
the method for subjecting the nucleic acid nanoball to the condensing treatment comprises at least one of steps a1) and a2):
   a1) adding a nucleic acid condensing agent to a system comprising the nucleic acid nanoball;
   a2) adjusting a pH of the system comprising the nucleic acid nanoball to 3-4.5;
the nucleic acid condensing agent comprises at least one of a multivalent salt, a basic peptide, a basic protein (e.g. histone, protamine), a polar solvent (e.g. an alcohol), a positively charged high molecular polymer (e.g. polyhistidine, polylysine, polyarginine, and polyornithine), a polyamine (e.g. spermine, spermidine, and putrescine), a polyammonium (e.g. polybrene (hexadimethrine bromide)), and a lipid (e.g. DOTAP, DC-Chol/DOPE, DOGS/DOPE, and DOTMA/DOPE).

During their research, the inventors learned that nucleic acids (e.g., DNA) are long-chain polymers composed of deoxynucleotides arranged in a repeated sequence. In polar solutions, the bases and phosphates in their structures carry a large amount of negative charge. By subjecting the nucleic acid (e.g., DNA) to a condensing treatment (e.g., adding a nucleic acid condensing agent (such as a multivalent salt, a basic peptide, a basic protein, a polar solvent (e.g., an alcohol), a positively charged high molecular polymer, a polyamine, a polyammonium, a lipid)) and/or adjusting the pH to 3-4.5, positively charged counterions are gathered around the nucleic acid (e.g., DNA). Through the electrostatic binding action between anions and cations, the negative charge on the surface of the nucleic acid (e.g., DNA) is neutralized by cations, thereby causing the morphology of the nucleic acid (e.g., DNA) to condense, which can subsequently alter the morphology of the nucleic acid nanoball (e.g., DNB), adjust the volume of the nucleic acid nanoball (e.g., DNB), and compress the nucleic acid nanoball (e.g., DNB), making the nucleic acid nanoball (e.g., DNB) more compact, smaller in morphology, and more uniform in size. Thus, the loading efficiency and quality of the nucleic acid nanoball (e.g., DNB) can be improved, making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality; at the same time, the detection signal can also be enhanced to meet the requirements of long-read sequencing.

Preferably, the method for subjecting the nucleic acid nanoball to the condensing treatment comprises: step a1).

Preferably, the method for subjecting the nucleic acid nanoball to the condensing treatment comprises: step a2).

Preferably, the method for subjecting the nucleic acid nanoball to the condensing treatment comprises: steps a1) and a2).

Preferably, the nucleic acid condensing agent in a1) comprises at least one of a multivalent salt, a polyamine, and a lipid; further preferably, the nucleic acid condensing agent in step a1) comprises a multivalent salt; still further preferably, the nucleic acid condensing agent in step a1) comprises a multivalent metal salt.

Preferably, the multivalent metal salt comprises at least one of a divalent metal salt and a trivalent metal salt; further preferably, the multivalent metal salt comprises at least one of a zinc salt, a calcium salt, a magnesium salt, a copper salt, and a cerium salt (e.g., it may be ZnCl₂, ZnSO₄, CaCl₂, MgCl₂, CuCl₂·2H₂O, CeCl₃·7H₂O); still further preferably, the multivalent metal salt comprises at least one of a copper salt, a cerium salt, a calcium salt, and a zinc salt. The use of the multivalent metal salt as the nucleic acid condensing agent can not only change the morphology of the nucleic acid nanoball (e.g., DNB), compress the nucleic acid nanoball (e.g., DNB), making the nucleic acid nanoball (e.g., DNB) more compact, smaller in morphology, and more uniform in size, thereby making the distribution of the nucleic acid nanoballs (e.g., DNB) loaded on the solid support (e.g., a chip) more uniform, with fewer empty sites and the size more uniform, the signal more concentrated, and less impact on adjacent sites, which can subsequently lower the Dup value, reduce empty sites on the chip, and improve sequencing quality; the detection signal can also be enhanced to meet the requirements of long-read sequencing; meanwhile, the adsorption capacity of the solid support (e.g., a chip) can also be improved.

Preferably, the polyamine comprises at least one of spermine and putrescine.

Preferably, the lipid comprises at least one of DOTAP and DC-Chol/DOPE.

Preferably, a final concentration of the nucleic acid condensing agent in a1) in the system is 0.001-1000 mM; further preferably 1-1000 mM; still further preferably 10-100 mM; still further preferably 12-80 mM.

Preferably, a final concentration of the zinc salt in the system is 1-1000 mM; further preferably 10-100 mM; still further preferably 20-80 mM.

Preferably, a final concentration of the calcium salt in the system is 1-1000 mM; further preferably 10-100 mM; still further preferably 12-20 mM.

Preferably, a final concentration of the copper salt in the system is 1-1000 mM; further preferably 10-100 mM; still further preferably 20-30 mM.

Preferably, a final concentration of the magnesium salt in the system is 1-1000 mM; further preferably 10-100 mM; still further preferably 20-30 mM.

Preferably, a final concentration of the cerium salt in the system is 1-1000 mM; further preferably 10-100 mM; still further preferably 20-30 mM.

Preferably, a final concentration of the polyamine in the system is 0.001-10 mM; further preferably 0.01-0.04 mM.

Preferably, a final concentration of the lipid in the system is 0.001-10 mM; further preferably 0.003-0.007 mM.

Preferably, the pH of the system in step a2) is 4-4.5; further preferably 4.2-4.4.

Preferably, the solid support comprises at least one of a latex bead, a dextran bead, a polystyrene surface, a polypropylene surface, a polyacrylamide gel, a gold surface, a glass surface, a chip, a sensor, an electrode, a silicon wafer; further preferably comprising a chip; still further preferably a high-density matrix chip.

Preferably, the solid support is planar, spherical, or porous.

Preferably, a material of the solid support includes materials such as glass, polyacrylamide-coated glass, ceramic, silica, silicon, quartz, various plastics, etc.

Preferably, the solid support is suitable for BGI sequencing platform.

Preferably, a maximum size of the nucleic acid nanoball (e.g., DNB) that a single site on the solid support can accommodate or attach is ≤5000nm, ≤2000nm, ≤1000nm, ≤700nm, ≤500nm, ≤300nm, or ≤100nm.

Preferably, a spacing between adjacent sites on the solid support is ≤5000nm, ≤2000nm, ≤1000nm, ≤700nm, ≤500nm, ≤300nm, or ≤100nm.

Preferably, the nucleic acid nanoball (e.g., DNB) is generated by Rolling Circle Amplification (RCA).

Preferably, the Rolling Circle Amplification (RCA) is a process that uses a single-stranded circular nucleic acid (e.g., DNA) as a template to massively replicate the nucleic acid (e.g., DNA).

Preferably, a method for preparing the single-stranded circular nucleic acid (e.g., DNA) is a conventional method in the art, namely circularizing a linear nucleic acid library.

Preferably, the circularization can be performed in the same reaction system as the rolling circle amplification (e.g., the one-step method for preparing DNB described in patent document CN115852493A), or performed in different systems (i.e., circularization performed in one reaction system, followed by rolling circle amplification in another reaction system; such as the commonly used method for preparing DNB in the art: circularization, enzymatic digestion, magnetic bead purification, denaturation/annealing, and rolling circle amplification).

Preferably, a method for constructing the linear nucleic acid library can be any method for constructing linear nucleic acid library, including but not limited to the methods for constructing linear nucleic acid library mentioned in the prior art.

Preferably, a nucleic acid used as a starting material for constructing the linear nucleic acid library can be any suitable DNA, RNA, or a nucleic acid complex of DNA-RNA, especially DNA, and particularly genomic DNA. The source of the nucleic acid is not limited; it can be nucleic acid from any biological source, such as nucleic acid from an animal, a plant, a microorganism, etc., especially nucleic acid from a mammal, particularly nucleic acid from a human, most preferably from human genomic DNA.

More specifically,
the nucleic acid can be isolated from a biological sample obtained from an individual (e.g., a test individual). The individual can be any living or non-living organism, including but not limited to humans, non-human animals, plants, bacteria, fungi, protists, or pathogens.

The nucleic acid can be isolated or obtained from any type of suitable biological sample. The nucleic acid can be isolated or obtained from a single cell, a plurality of cells (e.g., cultured cells), a cell culture medium, a conditioned medium, a tissue, an organ, or an organism (e.g., bacteria, yeast, etc.).

The nucleic acid can be isolated or obtained from an existing organism or animal. In some cases, the nucleic acid can be isolated or obtained from an extinct (or "ancient") organism or animal (e.g., an extinct mammal, an extinct mammal from the genus Homo, a paleontological fossil). In some cases, the nucleic acid can be obtained as part of a diagnostic assay.

In some cases, the nucleic acid can be isolated or obtained from a forensic sample or a specimen. The forensic sample or specimen may include any biological material containing the nucleic acid. For example, the forensic sample or specimen may include blood, semen, hair, skin, sweat, saliva, decomposed tissue, bone, nail scrapings, licked stamps/envelopes, sluff, contact DNA, razor residue, etc. The specimen can be formalin-fixed tissue and/or paraffin-embedded tissue.

A biological sample can be any sample isolated or obtained from an individual or part thereof (e.g., a human individual, a pregnant female, a cancer patient, a patient with an infection or infectious disease, a transplant recipient, a fetus, a tumor, an infected organ or tissue, a transplanted organ or tissue, a microbiome). In some embodiments, the biological sample is a cervical swab from an individual. The liquid sample or tissue sample from which nucleic acid is extracted can be cell-free (e.g., free of cells). In some embodiments, the biological sample may contain cellular components or cell remnants. In some embodiments, the biological sample may include fetal cells or cancer cells.

The biological sample can be a liquid sample. The liquid sample may contain extracellular nucleic acids (e.g., circulating cell-free DNA). Examples of the liquid sample include but are not limited to blood or blood products (e.g., serum, plasma, etc.), urine, cerebrospinal fluid, saliva, sputum, biopsy samples (e.g., liquid biopsy for cancer detection), the aforementioned liquid samples, analogs, or combinations thereof. In certain embodiments, the biological sample is a liquid biopsy, which generally refers to the evaluation of a liquid sample from an individual for the presence, absence, progression, or remission of a disease (e.g., cancer). The liquid biopsy can be used in conjunction with or as a substitute for an available biopsy (e.g., tumor biopsy). In some cases, extracellular nucleic acids are analyzed in the liquid biopsy.

The biological sample can be a tumor nucleic acid sample (i.e., a nucleic acid sample isolated from a tumor).

Preferably, the system comprising the nucleic acid nanoball in step a1) and/or step a2) further comprises a loading buffer for nucleic acid nanoball (e.g., DNB).

Preferably, the loading buffer for nucleic acid nanoball (e.g., DNB) is the loading buffer for nucleic acid nanoball according to the second aspect of the present invention.

Preferably, the single-stranded circular nucleic acid comprises at least one of single-stranded circular DNA libraries such as *E.coli*, PCR Free, and WGBS.

Preferably, the nucleic acid nanoball (e.g., DNB) is prepared using the rolling circle amplification technology of BGI sequencing platform.

Preferably, the BGI sequencing platform comprises MGISEQ-2000, MGISEQ-200, DNBSEQ-T7, DNBSEQ-T10, DNBSEQ-T20, DNBSEQ-G99, DNBSEQ-E25, etc.

Preferably, a rolling circle amplification time for the nucleic acid nanoball (e.g., DNB) is 20-120 min, thereby obtaining nucleic acid nanoball (e.g., DNB) with at least several hundred copies for sequencing, so as to improve the sequencing quality.

For the same RCA time, by subjecting the nucleic acid nanoball to the condensing treatment, the morphology of the nucleic acid nanoball (e.g., DNB) can be altered, the volume of the nucleic acid nanoball (e.g., DNB) can be adjusted, and the nucleic acid nanoball (e.g., DNB) can be compressed, thereby improving the loading efficiency and quality of the nucleic acid nanoball (e.g., DNB), so as to allow sequencing of the nucleic acid nanoball treated with the condensing treatment to achieve a lower Dup value, which subsequently improves the sequencing quality.

Preferably, the rolling circle amplification time for the nucleic acid nanoball (e.g., DNB) is 30-120 min.

Preferably, the rolling circle amplification time for the nucleic acid nanoball (e.g., DNB) is 40-120 min.

It is well known to those skilled in the art that within a certain time range, as the RCA time increases, the copy number of the nucleic acid nanoball (e.g., DNB) also increases. During gene sequencing, when the copy number of the nucleic acid nanoball (e.g., DNB) is high, the detection signal becomes stronger, which is beneficial for base calling and improves sequencing quality. However, an increased copy number of the nucleic acid nanoball (e.g., DNB) also leads to a larger volume of the nucleic acid nanoball (e.g., DNB), leading to greater mutual interference between the nucleic acid nanoballs (e.g., DNBs) when loaded onto the solid support (e.g., chip), issues such as more empty sites due to insufficient reactions, and non-uniform sizes of DNBs loaded onto the solid support (e.g., chip), thereby deteriorating the quality of gene sequencing. However, in the present invention, by subjecting the nucleic acid nanoball (e.g., DNB) having increased copy number and larger volume obtained with increased RCA time (by limiting the rolling circle amplification time to 30-120 min) to the condensing treatment (FIG. 2), the morphology of the nucleic acid nanoball (e.g., DNB) is altered, the volume of the nucleic acid nanoball (e.g., DNB) is adjusted, and the nucleic acid nanoball (e.g., DNB) is compressed, making the nucleic acid nanoball (e.g., DNB) having increased copy number and larger volume more compact and regular, and uniform in size, thereby improving the loading efficiency and quality of the nucleic acid nanoball (e.g., DNB), making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality of the nucleic acid nanoball (e.g., DNB) having increased copy number and larger volume obtained with increased RCA time (by limiting the rolling circle amplification time to 30-120 min). That is, the contradictory problem in the existing sequencing technology where the base signal to be enhanced by increasing the copy number is required, but the sequencing quality deteriorates, is solved.

Based on solving the contradictory problem of increasing the copy number to enhance the base signal but resulting in deteriorated sequencing quality, in the present invention, by simultaneously increasing the RCA time (by limiting the rolling circle amplification time to 30-120 min, also known as long RCA time) and subjecting the nucleic acid nanoball (e.g., DNB) to the condensing treatment having increased copy number and larger volume obtained with long RCA time, the morphology of the nucleic acid nanoball (e.g., DNB) is altered, the volume of the nucleic acid nanoball (e.g., DNB) is adjusted, and the nucleic acid nanoball (e.g., DNB) is compressed, making the nucleic acid nanoball (e.g., DNB) having increased copy number and larger volume obtained with long RCA time more compact in structure, and more uniform in size, thereby improving the loading efficiency and quality of the nucleic acid nanoball (e.g., DNB), making the distribution loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality of the nucleic acid nanoball (e.g., DNB) obtained with long RCA time; moreover, better sequencing quality is obtained compared to the nucleic acid nanoball (e.g., DNB) obtained with a short RCA time (15-25 min) but without the condensing treatment.

The fourth aspect of the present invention provides a sequencing method, comprising the following steps of:
1) adjusting a volume of a nucleic acid nanoball using the method according to the first aspect of the present invention; and
2) sequencing to obtain sequence information carried by the nucleic acid nanoball.

Preferably, step 1) may be performed in any one or more sequencing working processes of the sequencing method.

Preferably, the sequencing working processes include but are not limited to: loading process of nucleic acid nanoball, sequencing process of nucleic acid nanoball.

Preferably, when step 1) is performed during the loading process of nucleic acid nanoball, the system comprising the nucleic acid nanoball further comprises a loading buffer for nucleic acid nanoball.

Preferably, the loading buffer for nucleic acid nanoball comprises at least one of citrate buffer, MES buffer solution, and Tris hydrochloride buffer.

Preferably, when step 1) is performed during the sequencing process of nucleic acid nanoball, the system comprising the nucleic acid nanoball further comprises a sequencing reagent.

Preferably, the sequencing reagent comprises at least one of a dNTPs mixture, a nucleic acid polymerase mixture, an MDA reagent, and an MDA polymerase mixture.

Preferably, the method for subjecting the nucleic acid nanoball to the condensing treatment in step 1) during different sequencing working processes may be the same or different.

By adopting the method for adjusting the volume of nucleic acid nanoball (e.g., DNB) according to the first aspect of the present invention (subjecting the nucleic acid nanoball (e.g., DNB) to the condensing treatment) during the loading process of nucleic acid nanoball, the morphology of the nucleic acid nanoball (e.g., DNB) can be altered, the volume of the nucleic acid nanoball (e.g., DNB) can be adjusted, and the nucleic acid nanoball (e.g., DNB) can be compressed, making the nucleic acid nanoball (e.g., DNB) more compact, smaller in morphology, and more uniform in size, thereby improving the loading efficiency and quality of the nucleic acid nanoball (e.g., DNB), making the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality; at the same time, the detection signal can be enhanced to meet the requirements of long-read sequencing.

By adopting the method for adjusting the volume of nucleic acid nanoball (e.g., DNB) according to the first aspect of the present invention (subjecting the nucleic acid nanoball (e.g., DNB) to the condensing treatment) during the sequencing process of nucleic acid nanoball, the volume of the nucleic acid nanoball (e.g., DNB) can be adjusted (e.g., to maintain the morphology of the DNB), thereby improving the sequencing quality.

Preferably, the sequencing is performed using a BGI sequencing platform.

Preferably, the BGI sequencing platform comprises MGISEQ-2000, MGISEQ-200, DNBSEQ-T7, DNBSEQ-T10, DNBSEQ-T20, DNBSEQ-G99, DNBSEQ-E25, etc.

The fifth aspect of the present invention provides a kit comprising the reagent for adjusting the volume of nucleic acid nanoball (e.g., DNB) according to the second aspect of the present invention.

Preferably, the kit further comprises a preparation reagent for nucleic acid nanoball (e.g., DNB).

Preferably, the preparation reagent for nucleic acid nanoball (e.g., DNB) comprises at least one of TE buffer, nucleic acid nanoball (e.g., DNB) preparation buffer, a nucleic acid nanoball (e.g., DNB) polymerase mixture, and nucleic acid nanoball (e.g., DNB) termination buffer; further preferably comprising TE buffer, nucleic acid nanoball (e.g., DNB) preparation buffer, a nucleic acid nanoball (e.g., DNB) polymerase mixture, and nucleic acid nanoball (e.g., DNB) termination buffer.

Preferably, the TE buffer comprises Tris(hydroxymethyl)aminomethane, hydrochloric acid, and ethylenediaminetetraacetic acid.

Preferably, the nucleic acid nanoball (e.g., DNB) preparation buffer comprises ammonium sulfate, dithiothreitol, magnesium chloride, Tris(hydroxymethyl)aminomethane, and oligonucleotides.

Preferably, the nucleic acid nanoball (e.g., DNB) polymerase mixture comprises nucleic acid nanoball (e.g., DNB) polymerase mixture I and nucleic acid nanoball (e.g., DNB) polymerase mixture II; the nucleic acid nanoball (e.g., DNB) polymerase mixture I comprises: dNTPs, ammonium sulfate, dithiothreitol, magnesium chloride, Tris(hydroxymethyl)aminomethane, glycerol, and single-stranded binding protein; the nucleic acid nanoball (e.g., DNB) polymerase mixture II comprises: Tris(hydroxymethyl)aminomethane, potassium chloride, ethylenediaminetetraacetic acid, nucleic acid nanoball (e.g., DNB) polymerase, and glycerol.

Preferably, the nucleic acid nanoball (e.g., DNB) termination buffer comprises ethylenediaminetetraacetic acid.

The sixth aspect of the present invention provides use of the method for adjusting the volume of nucleic acid nanoball (e.g., DNB) according to the first aspect of the present invention, the reagent for adjusting the volume of nucleic acid nanoball (e.g., DNB) according to the second aspect of the present invention, and the kit according to the fifth aspect of the present invention in any one of f1) to f3):
f1) loading a nucleic acid nanoball (DNB);
f2) sequencing; and
f3) adjusting a volume of nucleic acid nanoball.

Preferably, the adjustment in the present invention refers to reduction.

The present invention is further described in detail below through specific examples.

It should be understood that these examples are only used to illustrate the present invention and are not used to limit the scope of the present invention.

Experimental methods in the following examples without specified specific conditions are generally carried out under conventional conditions, or according to the conditions recommended by the manufacturers. The materials, reagents, etc., used in the examples, unless otherwise specified, are reagents and materials obtained from commercial sources.

In the examples, the *Escherichia coli* (*E.coli*) and PCR-free libraries used were provided by MGI TECH. When using the MGISEQ-2000 sequencer for sequencing, equipment such as the sequencer and handheld loader were all produced and provided by Wuhan MGI TECH; the sequencing primers, sequencing reagents, Make DNB enzyme MIX I (DNB polymerase mixture I), Make DNB enzyme MIX II (DNB polymerase mixture II), stop run buffer, DNB loading buffer II (DLBII), and other reagents used were all from the MGISEQ-2000 standard PE100 sequencing reagent kit; and the sequencing carriers were provided by Wuhan MGI TECH. The ZnCl₂ (Sangon Biotech, A501003-0250), CaCl₂ (Sangon Biotech, A600506-0100), CuCl₂ (Sangon Biotech, A603090-0250), MgCl₂ (Sangon Biotech, A100288-0500), CeCl₃ (Sangon Biotech, A610054-0050) used were all purchased from MGI TECH.

### Example 1 Effect of Metal Ions on Dup Value

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 25 min.
2) Aqueous solutions of 1M ZnCl₂, CuCl₂, MgCl₂ and CeCl₃ were respectively added to the existing DLBII to obtain new DLBII solutions containing final concentrations of 80 mM ZnCl₂ (Experimental Group 3), CuCl₂ (Experimental Group 1), MgCl₂ (Experimental Group 2), and CeCl₃ (Experimental Group 4), respectively (the control group was left untreated).
3) The DNBs prepared in step 1) were mixed with the new DLBII obtained in step 2), respectively, so that final concentrations of ZnCl₂, CuCl₂, MgCl₂ and CeCl₃ in the mixed solutions were 20 mM, and were used for further testing (the workflow of the experiment was referred to the instruction manual of the gene sequencer (MGISEQ-2000), and single-end SE20 sequencing verification was performed on the MGISEQ-2000 platform).

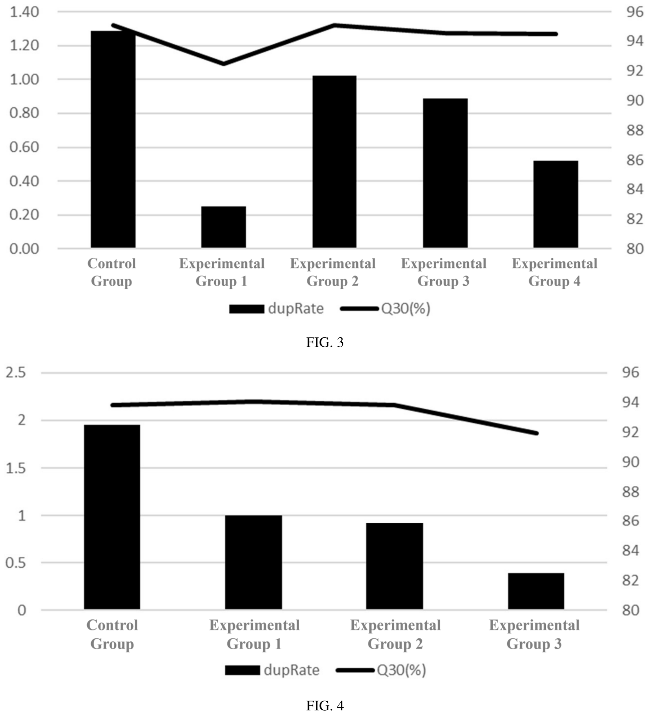

The SE20 sequencing results of the new DLBII containing metal ions were as shown in FIG. 3 and Table 1. Adding various metal ions that can cause DNB morphology to condense into the DLBII could alter DNB morphology, adjust DNB volume, and compress DNB, making its morphology smaller, thereby obtaining a lower Dup values by sequencing. Moreover, the ability to reduce the Dup value varied with different types of metal ions.

**Table 1. SE20 Sequencing Results of DNBs from PCR-free Library after Loaded via New DLBII Containing Metal Ions**

| **Sequencing Metric** | **Control Group** | **Experimental Group 1** | **Experimental Group 2** | **Experimental Group 3** | **Experimental Group 4** |
|---|---|---|---|---|---|
| Reference Species | PCR free | PCR free | PCR free | PCR free | PCR free |
| Sequencing Cycles | 20 | 20 | 20 | 20 | 20 |
| Filtered Read Size (M) | 546.96 | 507.99 | 533.63 | 544.47 | 533.36 |
| Filtered Q30 (%) | 95.1 | 92.48 | 95.1 | 94.56 | 94.52 |
| Sequencing Error Rate (%) | 0.46 | 0.75 | 0.24 | 0.27 | 0.29 |
| First Strand Sequencing Lead Proportion (%) | 0.11 | 0.11 | 0.1 | 0.1 | 0.11 |
| First Strand Sequencing Lag Proportion (%) | 0.15 | 0.17 | 0.14 | 0.14 | 0.15 |
| Duplicate Reads (%) | 1.29 | 0.25 | 1.02 | 0.89 | 0.52 |

### Example 2. Effect of DLBII pH on Dup Value

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 40 min.
2) DLBII with pH values of 4.7 (Control Group, pH of existing DLBII), 4.4 (Experimental Group 1), 4.3 (Experimental Group 2), and 4.2 (Experimental Group 3) were prepared respectively.
3) The DNBs prepared in step 1) were mixed with the DLBII obtained in step 2), and were used for further testing (the workflow of the experiment was referred to the instruction manual of the gene sequencer (MGISEQ-2000), and paired-end PE50 sequencing verification was performed on the MGISEQ-2000 platform).

The PE50 sequencing results of DLBII at different pH values were as shown in FIG. 4 and Table 2. Adjusting the pH of DLBII, especially lowering its pH, could alter DNB morphology, adjust DNB volume, and compress DNB, making its morphology smaller, thereby obtaining a lower Dup values by sequencing, and the lower the pH value, the smaller the Dup value.

**Table 2. PE50 Sequencing Results of DNBs from PCR-free Library after Loaded via New DLBII at Different pH Values**

| **Sequencing Metric** | **Control Group** | **Experimental Group 1** | **Experimental Group 2** | **Experimental Group 3** |
|---|---|---|---|---|
| Reference Species | PCR free | PCR free | PCR free | PCR free |
| Sequencing Cycles | 100 | 100 | 100 | 100 |
| Filtered Read Size | 418.16 | 394.73 | 406.66 | 368.8 |
| (M) | | | | |
| Filtered Q30 (%) | 93.84 | 94.04 | 93.85 | 91.95 |
| Sequencing Error Rate (%) | 0.28 | 0.27 | 0.28 | 0.28 |
| First Strand Sequencing Lead Proportion (%) | 0.11 | 0.11 | 0.11 | 0.11 |
| Second Strand Sequencing Lead Proportion (%) | 0.2 | 0.2 | 0.19 | 0.2 |
| First Strand Sequencing Lag Proportion (%) | 0.07 | 0.07 | 0.06 | 0.06 |
| Second Strand Sequencing Lag Proportion (%) | 0.13 | 0.08 | 0.08 | 0.08 |
| First Strand [AT] Discrepancy (%) | 1.01 | 0.7 | 0.42 | -0.05 |
| First Strand [CG] Discrepancy (%) | 0.49 | 0.76 | 1.09 | 1.68 |
| Second Strand [AT] Discrepancy (%) | 0.24 | 0.36 | 0.67 | 1.14 |
| Second Strand [CG] Discrepancy (%) | -0.15 | -0.26 | -0.57 | -0.98 |
| Duplicate Reads (%) | 1.95 | 1.00 | 0.92 | 0.39 |

### Example 3. Effect of Metal Ion Concentrations on Dup Value

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 25 min.
2) 1M CaCl₂ aqueous solution was added to the existing DLBII to obtain new DLBII containing final concentrations of 48 mM (Experimental Group 1) and 80 mM CaCl₂ (Experimental Group 2), respectively (the control group was left untreated).
3) The DNBs prepared in step 1) were mixed with the new DLBII obtained in step 2), so that final concentrations of CaCl₂ in the mixed solutions were 12 mM and 20 mM, respectively, and were used for further testing (the workflow of the experiment was referred to the instruction manual of the gene sequencer (MGISEQ-2000), and paired-end PE50 sequencing verification was performed on the MGISEQ-2000 platform).

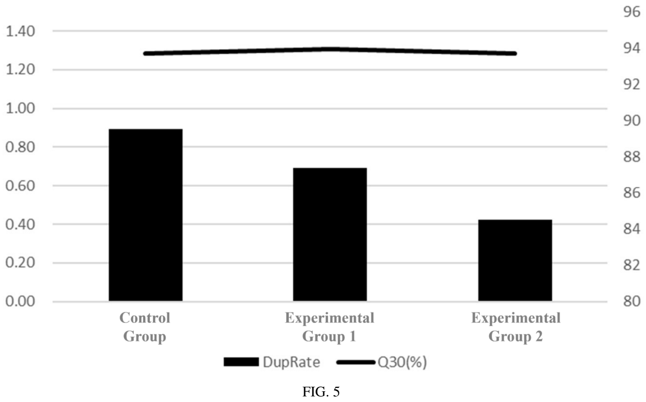

The PE50 sequencing results of DNBs obtained from the same RCA time after loaded via new DLBII containing different concentrations of CaCl₂ were as shown in FIG. 5 and Table 3. Adding various metal ions that can cause DNB morphology to condense into the DLBII could adjust (reduce) DNB volume, obtain a lower Dup values during sequencing. Moreover, as the metal ion concentration increases, the Dup value also decreases. To obtain better sequencing quality, when implementing the present technology of invention, the relationship between RCA time with different concentrations and types of metal ions needs to be fully considered, so as to obtain DNBs with morphology that better meets our requirements for improved sequencing quality.

**Table 3. PE50 Sequencing Results of DNBs from PCR-free Library after Loaded via New DLBII Containing Different Concentrations of Metal Ions**

| **Sequencing Metric** | **Control Group** | **Experimental Group 1** | **Experimental Group 2** |
|---|---|---|---|
| Reference Species | PCR free | PCR free | PCR free |
| Sequencing Cycles | 100 | 100 | 100 |
| Filtered Read Size (M) | 451.01 | 465.35 | 457.14 |
| Filtered Q30 (%) | 93.68 | 93.94 | 93.67 |
| Initial Signal-A | 385.36 | 381.74 | 417.56 |
| First Strand Sequencing Lead Proportion (%) | 0.09 | 0.09 | 0.09 |
| Second Strand Sequencing Lead Proportion (%) | 0.12 | 0.11 | 0.11 |
| First Strand Sequencing Lag Proportion (%) | 0.11 | 0.12 | 0.12 |
| Second Strand Sequencing Lag Proportion (%) | 0.16 | 0.16 | 0.16 |
| First Strand [AT] Discrepancy (%) | 1.31 | 1.27 | 1.14 |
| First Strand [CG] Discrepancy (%) | 0.1 | 0.28 | 0.47 |
| Second Strand [AT] Discrepancy (%) | -0.34 | -0.27 | -0.15 |
| Second Strand [CG] Discrepancy (%) | 0.39 | 0.33 | 0.21 |
| Duplicate Reads (%) | 0.89 | 0.69 | 0.42 |

### Example 4. Using Metal Ions to Improve Sequencing Quality of DNBs Obtained from RCA40min

1) An *E.coil* single-stranded circularized library was used with an input amount of 40 fmol. DNBs were prepared according to the MGISEQ-2000 DNB Sample Preparation and Loading Kit and the instruction manual. The RCA preparation time was 40 min.
2) 1M ZnCl₂ aqueous solution was added to the existing DLBII to obtain a new DLBII containing a final concentration of 80 mM ZnCl₂ (Experimental Group) (the control group was left untreated).
3) The DNBs prepared in step 1) were mixed with the new DLBII obtained in step 2), so that final concentration of ZnCl₂ in the mixed solution was 20 mM, and was used for further testing (the workflow of the experiment was referred to the instruction manual of the gene sequencer (MGISEQ-2000), and paired-end PE100 sequencing verification was performed on the MGISEQ-2000 platform).

The paired-end PE100 sequencing results of the new DLBII containing metal ions were as shown in FIG. 6 and Table 4. The Dup value of the experimental group was smaller than that of the control group, indicating that use of the new DLBII containing metal ions to load DNBs could adjust (reduce) DNB volume, so that DNBs with long RCA times obtained smaller Dup values. Furthermore, based on the signals, use of the new DLBII containing metal ions to load DNBs could improve the loading of large DNBs, making their arrangement more orderly and regular, mutual interference between DNBs reduced, the signals more concentrated, thereby strengthening the sequencing signals and mitigating the problem of rapid sequencing quality decline. Judging from sequencing metrics such as filtered Q30, [AT] discrepancy, and average sequencing coverage depth, use of the new DLBII containing metal ions to load DNBs mitigated the loading problem of large DNBs, so that better sequencing quality could be obtained in high-throughput sequencing.

**Table 4. PE100 Sequencing Results of DNBs from E.coli Library after Loaded via New DLBII Containing Metal Ions**

| **Sequencing Metric** | **Control Group** | **Experimental Group** |
|---|---|---|
| Reference Species | *E.coli* | *E.coli* |
| Sequencing Cycles | 200 | 200 |
| Filtered Read Size (M) | 424.76 | 426.93 |
| Filtered Q30 (%) | 94.175 | 95.2 |
| Initial Signal-A | 635.147278 | 678.738161 |
| First Strand Sequencing Lead Proportion (%) | 0.11 | 0.11 |
| Second Strand Sequencing Lead Proportion (%) | 0.13 | 0.13 |
| First Strand Sequencing Lag Proportion (%) | 0.12 | 0.12 |
| Second Strand Sequencing Lag Proportion (%) | 0.13 | 0.13 |
| First Strand [AT] Discrepancy (%) | 0.77 | 0.2 |
| First Strand [CG] Discrepancy (%) | 0.2 | 0.31 |
| Second Strand [AT] Discrepancy (%) | 0.27 | 0.84 |
| Second Strand [CG] Discrepancy (%) | 0.18 | 0.35 |
| Average Sequencing Coverage Depth (X) | 14.94 | 15.13 |
| Duplicate Reads (%) | 1.85 | 0.65 |
| Mapping Rate (%) | 99.67 | 99.7 |

The above sequenced chips were characterized by atomic force microscopy to observe DNB morphology. The results were as shown in FIG. 7. The DNB morphology in the control group was non-uniform in size and diverse in morphology, with significant variation in DNB heights. Compared with the control group, the DNBs in the experimental group were more compact in morphology, more uniform in size, more orderly in arrangement, with fewer empty sites, and less variation in height. This indicated that use of the new DLBII containing metal ions to load DNBs altered DNB morphology, making DNBs more compact, more uniform in size, more orderly in arrangement, and with fewer empty sites.

### Example 5. Using Metal Ions to Improve Sequencing Quality of DNBs Obtained from RCA120min

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 120 min.
2) 1M ZnCl₂ aqueous solution was added to the existing DLBII to obtain new DLBII containing final concentrations of 160 mM (Experimental Group 1) and 320 mM (Experimental Group 2), respectively (the control group was left untreated).
3) The DNBs prepared in step 1) were mixed with the new DLBII obtained in step 2), so that the final concentrations of ZnCl₂ in the mixed solution were 40 mM and 80 mM, respectively, and were used for further testing (the workflow of the experiment was referred to the instruction manual of the gene sequencer (MGISEQ-2000), and paired-end PE100 sequencing verification was performed on the MGISEQ-2000 platform).

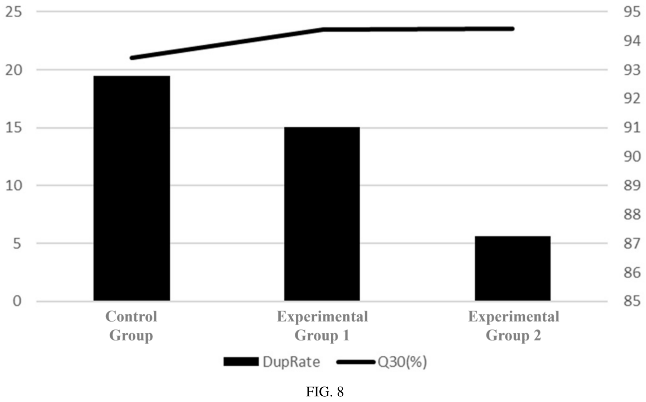

The PE100 sequencing results of DNBs obtained from extended RCA time of 120 min and loaded via new DLBII containing different concentrations of ZnCl₂ were as shown in FIG. 8 and Table 5. DNBs obtained by greatly extending the RCA time to 120 min were larger than those by a conventional RCA time of 25 min. By using the new DLBII of the present invention and employing higher concentrations of ZnCl₂, the DNB volume could be adjusted (reduced), resulting in smaller Dup values and higher sequencing quality. This indicated that the new DLBII of the present invention could further improve the loading and sequencing quality of DNBs from longer RCA times by adjusting the concentration of the added condensing agent.

**Table 5. PE100 Sequencing Results of DNBs from PCR-free Library after Loaded via New DLBII Containing Different Concentrations of ZnCl₂**

| **Sequencing Metric** | **Control Group** | **Experimental Group 1** | **Experimental Group 2** |
|---|---|---|---|
| Reference Species | PCR free | PCR free | PCR free |
| Sequencing Cycles | 200 | 200 | 200 |
| Filtered Read Size (M) | 387.89 | 408.37 | 421.08 |
| Filtered Q30 (%) | 93.42 | 94.37 | 94.41 |
| Initial Signal-A | 1242.617716 | 1352.071546 | 1813.355092 |
| First Strand Sequencing Lead Proportion (%) | 0.11 | 0.11 | 0.12 |
| Second Strand Sequencing Lead Proportion (%) | 0.14 | 0.15 | 0.14 |
| First Strand Sequencing Lag Proportion (%) | 0.05 | 0.05 | 0.07 |
| Second Strand Sequencing Lag Proportion (%) | 0.06 | 0.06 | 0.08 |
| First Strand [AT] Discrepancy (%) | 1.09 | 0.42 | 0.08 |
| First Strand [CG] Discrepancy (%) | -0.13 | 0.63 | 1.06 |
| Second Strand [AT] Discrepancy (%) | 0.61 | 0.68 | 0.82 |
| Second Strand [CG] Discrepancy (%) | -0.49 | -0.61 | -0.75 |
| Duplicate Reads (%) | 19.4711 | 15.0768 | 5.599 |
| Error Rate (%) | 0.36 | 0.2 | 0.2 |

### Example 6. Sequencing Quality of DNBs Obtained from Treatment with Different RCA times Using Metal Ion

1) A PCR-free single-stranded circularized DNA library was used with an input amount of 6 ng. DNBs were prepared according to the MGISEQ-2000 DNB Sample Preparation and Loading Kit and the instruction manual. The RCA preparation times were 25 min and 30 min, respectively. The concentration of the DNBs prepared above was detected using ssDNA Qubit quantification detection reagent and was recorded as C1. The experimental results were as shown in Table 6. As the RCA time increased, the DNB copy number increased, the DNB concentration became higher, and the obtained DNB morphology also became larger.
Table 6. DNB Concentrations Obtained from PCR-free Library after Different RCA Times

| RCA Time | C1 (ng/µL) |
|---|---|
| 25 min | 14.8 |
| 30 min | 19.9 |

2) 1M ZnCl₂ aqueous solution was added to the existing DLBII to obtain a new DLBII containing a final concentration of 80 mM ZnCl₂ (the control group was left untreated).
3) The DNBs prepared in step 1) were mixed with the new DLBII obtained in step 2) (wherein, the control group was: RCA 25min + DLBII (untreated DLBII, Control Group); Experimental Group 1 was: RCA 25min + new DLBII containing 80 mM ZnCl₂ (a final concentration of ZnCl₂ in the mixed solution was 20 mM); Experimental Group 2 was: RCA 30min + new DLBII containing 80 mM ZnCl₂ (a final concentration of ZnCl₂ in the mixed solution was 20 mM)), and were used for further testing (the workflow of the experiment was referred to the instruction manual of the gene sequencer (MGISEQ-2000), and paired-end PE50 sequencing verification was performed on the MGISEQ-2000 platform).

The results were as shown in FIG. 9 and Table 7. By comparing Experimental Group 1 with the Control Group, it was obvious that for the same RCA time, using DLBII containing metal ions (ZnCl₂) to load DNBs could alter DNB morphology, adjust (reduce) DNB volume, resulting in a smaller Dup value. By comparing Experimental Group 2 with the Control Group, it was obvious that with the increase of RCA time, even if the DNB copy number increased and the DNB morphology became larger, using DLBII containing metal ions (ZnCl₂) to load DNBs could still alter DNB morphology, adjust (reduce) DNB volume, lower the Dup value and error rate, and improve Q30 and sequencing signals, etc., resulting in better sequencing quality than the control group. This indicated that using DLBII containing metal ions (ZnCl₂) to load DNBs was suitable for improving the loading of large DNBs obtained from long RCA times.

**Table 7. PE50 Sequencing Results of DNBs from PCR-free Library after Loaded via New DLBII Containing Metal Ions**

| **Sequencing Metric** | **Control Group** | **Experimental Group 1** | **Experimental Group 2** |
|---|---|---|---|
| Reference Species | PCR free | PCR free | PCR free |
| Sequencing Cycles | 100 | 100 | 100 |
| Filtered Read Size (M) | 452.96 | 393.93 | 448.43 |
| Filtered Q30 (%) | 93.38 | 91.78 | 94.08 |
| Initial Signal-A | 363.0277 | 357.384 | 430.5205 |
| First Strand Sequencing Lead Proportion (%) | 0.11 | 0.12 | 0.12 |
| Second Strand Sequencing Lead Proportion (%) | 0.16 | 0.16 | 0.17 |
| First Strand Sequencing Lag Proportion (%) | 0.08 | 0.08 | 0.06 |
| Second Strand Sequencing Lag Proportion (%) | 0.1 | 0.1 | 0.07 |
| First Strand [AT] Discrepancy (%) | 1 | 0.12 | 0.48 |
| First Strand [CG] Discrepancy (%) | 0.45 | 1.53 | 1.09 |
| Second Strand [AT] Discrepancy (%) | 0.04 | 0.54 | 0.12 |
| Second Strand [CG] Discrepancy (%) | 0.01 | -0.44 | -0.06 |
| Duplicate Reads (%) | 0.219 | 0.077 | 0.165 |
| Error Rate (%) | 0.31 | 0.56 | 0.26 |

It could be seen that the method for improving the loading efficiency and quality of nucleic acid nanoballs (e.g., DNBs) involved in the present invention (causing DNB to condense, (e.g., by adding a nucleic acid condensing agent to the solution and/or adjusting the pH value of the solution)) was more suitable for loading large DNBs with increased RCA time and higher copy number. It improved sequencing signals while reducing the Dup value. The combination of these two effects improved the sequencing quality of large nucleic acid nanoballs and was beneficial for long-read sequencing.

### Example 7. Effect of Polyamines on Dup Value

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 25 min.
2) Spermine and putrescine were added to the existing DLBII to obtain new DLBII containing spermine and putrescine, respectively (the control group was left untreated).
3) The DNBs prepared in step 1) were mixed with the new DLBII obtained in step 2), respectively, so that final concentrations of spermine and putrescine in the mixed solution were 0.003 mg/mL (converted to molar concentrations of approximately 0.015 mM and 0.034 mM, respectively), and were used for further testing (the workflow of the experiment was referred to the instruction manual of the gene sequencer (MGISEQ-2000), and single-end SE20 sequencing verification was performed on the MGISEQ-2000 platform).

The SE20 sequencing results of the new DLBII containing polyamines in the present example were similar to the SE20 sequencing results of the new DLBII containing metal ions in Example 1. Adding polyamines (e.g., spermine or putrescine) that can cause DNB morphology to condense into DLBII could alter DNB morphology, adjust DNB volume, compress DNBs, making their morphology smaller, thereby obtaining lower Dup values by sequencing.

### Example 8. Effect of Lipids on Dup Value

1) A PCR-free single-stranded circularized DNA library was used with an input amount of 6 ng. DNBs were prepared according to the MGISEQ-2000 DNB Sample Preparation and Loading Kit and the instruction manual. The RCA preparation time was 25 min.
2) DOTAP and DC-Chol/DOPE (molar ratio 1:1) were added to the existing DLBII to obtain new DLBII containing DOTAP and DC-Chol/DOPE, respectively (the control group was left untreated).
3) The DNBs prepared in step 1) were mixed with the new DLBII obtained in step 2), respectively, so that final concentrations of DOTAP and DC-Chol/DOPE in the mixed solution were 0.005 mg/mL (converted to molar concentrations of approximately 0.00646 mM and 0.0039 mM, respectively), and were used for further testing (the workflow of the experiment was referred to the instruction manual of the gene sequencer (MGISEQ-2000), and single-end SE20 sequencing verification was performed on the MGISEQ-2000 platform).

The SE20 sequencing results of the new DLBII containing lipids in the present example were similar to the SE20 sequencing results of the new DLBII containing metal ions in Example 1. Adding lipids (e.g., DOTAP or DC-Chol/DOPE) that can cause DNB morphology to condense into DLBII could alter DNB morphology, adjust DNB volume, compress DNBs, making their morphology smaller, thereby obtaining lower Dup values by sequencing.

It could be seen that the method for improving the loading efficiency and quality of nucleic acid nanoballs (e.g., DNBs) involved in the present invention (causing DNB to condense, (e.g., by adding a nucleic acid condensing agent to the solution and/or adjusting the pH value of the solution)) could make the distribution of the nucleic acid nanoballs (e.g., DNB) loaded on the solid support (e.g., a chip) more uniform, with fewer empty sites and the size more uniform, the signal more concentrated, and less impact on adjacent sites, thereby lowering the Dup value, reducing empty sites on the chip, and improving sequencing quality; it was particularly suitable for loading large DNBs with increased RCA time and higher copy numbers, improving sequencing signals while reducing the Dup value. The combination of these two effects improved the sequencing quality of large nucleic acid nanoballs and was beneficial for long-read sequencing.

Furthermore, the condensing treatment could also be performed during the sequencing process (e.g., by adding a nucleic acid condensing agent to the sequencing reagent and/or adjusting the pH of the sequencing reagent) to further adjust the DNB volume (e.g., to maintain the morphology of the DNB), and further improving sequencing quality.

That is, the present invention provides a method for adjusting (reducing) the volume of nucleic acid nanoballs (DNBs), which can be used in any situation requiring adjustment (reduction) of DNB volume (which can be in any one or more sequencing working processes, such as the loading process of nucleic acid nanoball (e.g., DNB), and the sequencing process after loading); wherein, by causing the nucleic acid nanoballs (e.g., DNB) to condense during the loading process of nucleic acid nanoballs (e.g., by adding a nucleic acid condensing agent to the solution and/or adjusting the pH value of the solution), the effect of adjusting (reducing) the volume of nucleic acid nanoballs is achieved, making the nucleic acid nanoballs more compact, smaller in morphology, and more uniform in size, thereby improving the loading efficiency and quality of the nucleic acid nanoballs, making the distribution of the nucleic acid nanoballs loaded on the chip more uniform, with fewer empty sites and the sizes more uniform, the signals more concentrated, and less impact on adjacent sites, thereby lowering the Dup values, reducing empty sites on the chip, and improving sequencing quality. In addition, it was more suitable for loading large nucleic acid nanoballs (DNBs) with increased RCA time and higher copy numbers, improving sequencing signals while reducing the Dup value. The combination of these two effects meets the requirements for long-read sequencing. Moreover, the condensing treatment can be performed during the sequencing process to further adjust the volume of nucleic acid nanoballs (DNBs) (e.g., to maintain the morphology of the DNB), and further improving sequencing quality.

The above examples are preferred embodiments of the present invention, but the embodiments of the present invention are not limited by the above examples. Any other changes, modifications, substitutions, combinations, or simplifications made without departing from the spirit and principles of the present invention should be considered as equivalent replacement methods and are included within the scope of protection of the present invention.

## Claims

1. A method for adjusting a volume of a nucleic acid nanoball, comprising subjecting the nucleic acid nanoball to a condensing treatment to obtain a volume-compressed nucleic acid nanoball;
wherein a method for subjecting the nucleic acid nanoball to the condensing treatment comprises at least one of steps a1) and a2):
a1) adding a nucleic acid condensing agent to a system comprising the nucleic acid nanoball; and
a2) adjusting a pH of the system comprising the nucleic acid nanoball to 3-4.5;
wherein the nucleic acid condensing agent comprises at least one of a multivalent salt, a basic peptide, a basic protein, a polar solvent, a positively charged high molecular polymer, a polyamine, a polyammonium, and a lipid.

2. The method according to claim 1, wherein:
the method for subjecting the nucleic acid nanoball to the condensing treatment comprises: steps a1) and 2);
preferably, the basic protein comprises at least one of histone and protamine; and/or
the polar solvent comprises an alcohol; and/or
the positively charged high molecular polymer comprises at least one of polyhistidine, polylysine, polyarginine, and polyornithine; and/or
the polyamine comprises at least one of spermine, spermidine, and putrescine; the polyamine preferably comprises at least one of spermine and putrescine; and/or
the polyammonium comprises hexadimethrine bromide; and/or
the lipid comprises at least one of DOTAP, DC-Chol/DOPE, DOGS/DOPE, and DOTMA/DOPE; the lipid preferably comprises at least one of DOTAP and DC-Chol/DOPE.

3. The method according to claim 1, wherein:
the nucleic acid condensing agent in step a1) comprises at least one of a multivalent salt, a polyamine, and a lipid;
preferably, the nucleic acid condensing agent in step a1) comprises a multivalent metal salt;
preferably, the multivalent metal salt comprises at least one of a divalent metal salt and a trivalent metal salt;
preferably, the multivalent metal salt comprises at least one of a zinc salt, a calcium salt, a magnesium salt, a copper salt, and a cerium salt.

4. The method according to any one of claims 1-3, wherein:
in step a1), a final concentration of the nucleic acid condensing agent in the system is 0.001-1000 mM; and/or
in step a2), the pH of the system is 4-4.5;
preferably, wherein in step a1), the final concentration of the nucleic acid condensing agent in the system is 1-1000 mM;
preferably, in step a1), the final concentration of the nucleic acid condensing agent in the system is 10-100 mM;
preferably, in step a1), the final concentration of the nucleic acid condensing agent in the system is 12-80 mM;
Preferably, in step a2), the pH of the system is 4.2-4.4.

5. A reagent for adjusting a volume of a nucleic acid nanoball, wherein the reagent for adjusting the volume of the nucleic acid nanoball comprises at least one of b1) to b2):
b1) the reagent for adjusting the volume of the nucleic acid nanoball comprises a nucleic acid condensing agent; and
b2) a pH of the reagent for adjusting the volume of the nucleic acid nanoball is 3-4.5;
wherein the nucleic acid condensing agent comprises at least one of a multivalent salt, a basic peptide, a basic protein, a polar solvent, a positively charged high molecular polymer, a polyamine, a polyammonium, and a lipid.

6. The reagent for adjusting the volume of the nucleic acid nanoball according to claim 5, wherein:
the reagent for adjusting the volume of the nucleic acid nanoball further comprises a loading buffer for the nucleic acid nanoball, or a sequencing reagent;
preferably, the basic protein comprises at least one of histone and protamine; and/or
the polar solvent comprises an alcohol; and/or
the positively charged high molecular polymer comprises at least one of polyhistidine, polylysine, polyarginine, and polyornithine; and/or
the polyamine comprises at least one of spermine, spermidine, and putrescine; the polyamine preferably comprises at least one of spermine and putrescine; and/or
the polyammonium comprises hexadimethrine bromide; and/or
the lipid comprises at least one of DOTAP, DC-Chol/DOPE, DOGS/DOPE, and DOTMA/DOPE; the lipid preferably comprises at least one of DOTAP and DC-Chol/DOPE.

7. The reagent for adjusting the volume of the nucleic acid nanoball according to claim 6, wherein:
the reagent for adjusting the volume of the nucleic acid nanoball comprises b1) and b2).

8. The reagent for adjusting the volume of the nucleic acid nanoball according to any one of claims 5-7, wherein:
in b1), the nucleic acid condensing agent comprises at least one of a multivalent salt, a polyamine, and a lipid; and/or
in b2), the pH of the reagent is 4-4.5;
preferably, in b1), the nucleic acid condensing agent comprises a multivalent metal salt;
preferably, the multivalent metal salt comprises at least one of a divalent metal salt and a trivalent metal salt;
preferably, the multivalent metal salt comprises at least one of a zinc salt, a calcium salt, a magnesium salt, a copper salt, and a cerium salt;
preferably, in b2), the pH of the reagent is 4.2-4.4.

9. The reagent for adjusting the volume of the nucleic acid nanoball according to claim 8, wherein:
a method for using the reagent for adjusting the volume of the nucleic acid nanoball comprises: mixing the reagent for adjusting the volume of the nucleic acid nanoball with a nucleic acid nanoball to obtain a system for adjusting the volume of the nucleic acid nanoball;
and wherein the system satisfies at least one of c1) to c2):
c1) a final concentration of the nucleic acid condensing agent in the system for adjusting the volume of the nucleic acid nanoball is 0.001-1000 mM; and
c2) a pH of the system for adjusting the volume of the nucleic acid nanoball is 3-4.5;
preferably, in c1), the final concentration of the nucleic acid condensing agent in the system for adjusting the volume of the nucleic acid nanoball is 1-1000 mM; further preferably 10-100 mM; still further preferably 12-80 mM;
preferably, in c2), the pH of the system for adjusting the volume of the nucleic acid nanoball is 4-4.5; further preferably 4.2-4.4;
preferably, the loading buffer for the nucleic acid nanoball comprises at least one of a citrate buffer, a MES buffer solution, and a Tris hydrochloride buffer;
preferably, the sequencing reagent comprises at least one of a dNTPs mixture, a nucleic acid polymerase mixture, an MDA reagent, and an MDA polymerase mixture.

10. A method for improving loading efficiency and quality of a nucleic acid nanoball, wherein the method for adjusting the volume of the nucleic acid nanoball according to any one of claims 1-4, is used during loading process of the nucleic acid nanoball.

11. The method according to claim 10, wherein:
the method for improving loading efficiency and quality of the nucleic acid nanoball comprises the following steps:
subjecting a nucleic acid nanoball to a condensing treatment to obtain a volume-compressed nucleic acid nanoball; and
loading the volume-compressed nucleic acid nanoball onto a solid support;
wherein a method for subjecting the nucleic acid nanoball to the condensing treatment is the method for subjecting the nucleic acid nanoball to the condensing treatment according to any one of claims 1-4;
preferably, the solid support comprises at least one of a latex bead, a dextran bead, a polystyrene surface, a polypropylene surface, a polyacrylamide gel, a gold surface, a glass surface, a chip, a sensor, an electrode, and a silicon wafer.

12. The method according to claim 10 or 11, wherein:
the nucleic acid nanoball is prepared by rolling circle amplification;
preferably, a rolling circle amplification time for the nucleic acid nanoball is 20-120 min;
preferably, the rolling circle amplification time for the nucleic acid nanoball is 30-120 min;
preferably, the rolling circle amplification time for the nucleic acid nanoball is 40-120 min;
preferably, the system comprising the nucleic acid nanoball in step a1) and/or step a2) further comprises a loading buffer for nucleic acid nanoball;
preferably, the loading buffer for nucleic acid nanoball comprises at least one of a citrate buffer, a MES buffer solution, and a Tris hydrochloride buffer.

13. A sequencing method, comprising the following steps of:
1) adjusting a volume of a nucleic acid nanoball using the method according to any one of claims 1-4; and
2) sequencing to obtain sequence information carried by the nucleic acid nanoball.

14. The method according to claim 13, wherein:
step 1) may be performed in any one or more sequencing working processes of the sequencing method;
preferably, the sequencing working processes comprise but are not limited to loading process of nucleic acid nanoball and sequencing process of nucleic acid nanoball;
preferably, when step 1) is performed during the loading process of nucleic acid nanoball, the system comprising the nucleic acid nanoball further comprises a loading buffer for nucleic acid nanoball;
preferably, the loading buffer for nucleic acid nanoball comprises at least one of a citrate buffer, a MES buffer solution, and a Tris hydrochloride buffer;
preferably, when step 1) is performed during the sequencing process of nucleic acid nanoball, the system comprising the nucleic acid nanoball further comprises a sequencing reagent;
preferably, the sequencing reagent comprises at least one of a dNTPs mixture, a nucleic acid polymerase mixture, an MDA reagent, and an MDA polymerase mixture.

15. A kit comprising the reagent for adjusting the volume of the nucleic acid nanoball according to any one of claims 5-9;
wherein the kit further comprises a preparation reagent for nucleic acid nanoball;
preferably, the preparation reagent for nucleic acid nanoball comprises at least one of a TE buffer, a nucleic acid nanoball preparation buffer, a nucleic acid nanoball polymerase mixture, and a nucleic acid nanoball termination buffer.
